Europäisches Patentamt
European Patent Office
Office européen des brevets

(11) Veröffentlichungsnummer: **0 237 917 A2**

(12) **EUROPÄISCHE PATENTANMELDUNG**

(21) Anmeldenummer: 87103391.6

(22) Anmeldetag: 10.03.87

(51) Int. Cl.³: **C 07 D 249/08**
C 07 D 233/56, C 07 D 409/1- 2
C 07 D 405/12, C 07 D 417/1- 2
C 07 D 403/12, C 07 D 401/1- 2
A 61 K 31/41, A 61 K 31/415
A 01 N 43/653, A 01 N 43/50

(30) Priorität: 15.03.86 DE 3608792

(43) Veröffentlichungstag der Anmeldung: 23.09.87 Patentblatt 87/39

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Blume, Ernst, Dr.
Rossertstrasse 20
D-6239 Kriftel(DE)

(72) Erfinder: Schaper, Wolfgang, Dr.
Rauenthaler Weg 18
D-6000 Frankfurt am Main(DE)

(72) Erfinder: Ehrhardt, Heinz, Dr.
Bergstrasse 21
D-8901 Rehling(DE)

(72) Erfinder: Raether, Wolfgang, Dr.
Falkensteinstrasse 6
D-6072 Dreieich(DE)

(72) Erfinder: Dittmar, Walter, Dr.
Uhlandstrasse 10
D-6238 Hofheim am Taunus(DE)

(72) Erfinder: Hänel, Heinz, Dr.
Tannenwaldallee 80
D-6380 Bad Homburg(DE)

(72) Erfinder: Sachse, Burkhard, Dr.
An der Ziegelei 30
D-6233 Kelkheim (Taunus)(DE)

(54) **1,1-Disubstituierte Cyclopropanderivate, Verfahren zu ihrer Herstellung und ihre Verwendung.**

(57) Verbindungen I

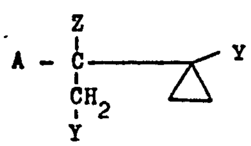

I,

mit A gleich t-Butyl oder einer Vielzahl von (substituierten) Aromaten; Y gleich Azolyl; oder Y gleich $-X-R^1$ mit X = Sauerstoff oder Schwefel und $R^1$ gleich (Cyclo)-Alkyl, (heterocyclisches) Aryl; oder Y gleich 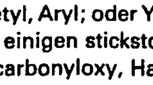 mit $R^2$ gleich (Cyclo)-Alkyl, Acetyl, Aryl; oder Y gleich $-NR^3R^4$ mit $R^3/R^4$ Alkyl; oder Y gleich einigen stickstoffhaltigen Heterocyclen; Z gleich OH, Alkylcarbonyloxy, Hal, Alkoxy, Benzyloxy; und deren Salze sind wirksame Antimykotika bzw. Fungizide.

Sie werden erhalten durch Umsetzung von II

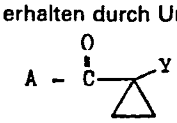

II

mit $(CH_3)_2$ $SCH_2$
|
$(O)_p$

III und weitere Reaktion der dabei erhaltenen Verbindung IV

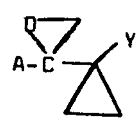

, mit einem Nucleophil YM (M = Wasserstoff oder Metalläquivalent).

Die dabei resultierenden Verbindungen I (mit Z = OH) können acyliert, alkyliert oder in I mit Z = Hal überführt werden.

EP 0 237 917 A2

HOECHST AKTIENGESELLSCHAFT HOE 86/F 060 Dr.v.F./St

1,1-Disubstituierte Cyclopropanderivate, Verfahren zu ihrer Herstellung und ihre Verwendung

Die Erfindung betrifft 1,1-disubstituierte Cyclopropanderivate, Verfahren zu ihrer Herstellung, sowie solche Verbindungen enthaltende pharmazeutische Zusammensetzungen und ihre Verwendung als Pharmazeutika, insbesondere als Antimykotika und Pflanzenschutzmittel, z.B. als Fungizide und Wachstumsregulatoren.

In der Patentschrift DE-A-34 33 553 werden 1-Aryl-1-cyclopropyl-2-azolyl-ethanole und in EP-A 0 164 246 Bis-Triazole als Antimykotika und Fungizide beschrieben; ihre Wirkung und Verträglichkeit ist jedoch nicht ganz befriedigend.

Es wurde nun gefunden, daß die erfindungsgemäßen Verbindungen, die sich von den oben erwähnten Verbindungen wesentlich durch die Art der Substituenten am Cyclopropanring unterscheiden, sehr gute antimikrobielle, insbesondere antimykotische Eigenschaften aufweisen. Sie sind deshalb zur Bekämpfung von Pilzen bei Mensch und Tier geeignet. Durch fungizide und wachstumsregulierende Eigenschaften sind sie auch als Pflanzenschutzmittel geeignet.

Die Erfindung betrifft daher Cyclopropanderivate der Formel (I)

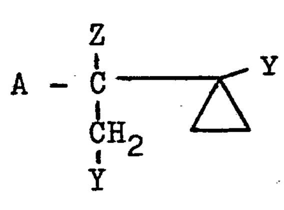

worin bedeuten:

A t-Butyl, Phenyl, Biphenyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-tetrahydronaphthyl, Indanyl, Fluorenyl, Thienyl, Furyl, Pyridyl, Isoxazolyl, Pyrazolyl, Benzofuryl, Benzothienyl,

wobei die genannten Ringsysteme unsubstituiert oder mit 1-3 Substituenten, die gleich oder verschieden sind, substituiert sind, welche F, Cl, Br, J, $(C_1-C_8)$-Alkyl, geradkettig oder verzweigt und unsubstituiert oder mit 1-9 F- oder Cl-Atomen substituiert, $(C_3-C_8)$-Cycloalkyl, $(C_1-C_8)$-Alkoxy, geradkettig oder verzweigt, und unsubstituiert oder mit 1-9 F- oder Cl-Atomen substituiert, $(C_3-C_8)$Cycloalkyl-$(C_1-C_4)$Alkyl, $(C_1-C_8)$Alkylthio, $(C_1-C_8)$-Alkylsulfinyl- und sulfonyl, $NO_2$, CN sind;

Y

1) 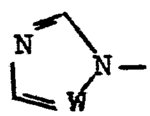

mit W= CH oder N

2) - X - $R^1$

mit X = -O-, -S-, S=O, $SO_2$ und

$R^1$= $(C_1-C_{12})$-Alkyl (geradkettig oder verzweigt, unsubstituiert oder durch 1 bis 3 F-, Cl- oder Br-Atome oder $CH_3$-Gruppen substituiert), $(C_2-C_{20})$-Alkenyl (geradkettig oder verzweigt; ein- oder mehrfach ungesättigt, in Form der reinen e- oder z-Isomeren oder der e/z-Diastereomerengemische, unsubstituiert oder durch 1 bis 3 F-, Cl- oder Br-Atome oder $CH_3O$-Gruppen substituiert), $(C_2-C_{20})$-Alkinyl (geradkettig oder verzweigt; unsubstituiert oder durch 1 bis 3 F-, Cl- oder Br-Atome oder $CH_3O$-Gruppen substituiert), $(C_2-C_{20})$Alkeninyl (geradkettig oder verzweigt; ein- oder mehrfach ungesättigt, in Form der reinen e- oder z-Isomeren oder der

e/z-Diastereomerengemische, unsubstituiert oder durch 1 bis 3 F-, Cl- oder Br-Atome oder $CH_3O$-Gruppen substituiert), $(C_3-C_8)$-Cycloalkyl, $(C_3-C_8)$Cycloalkyl-$(C_1-C_4)$-alkyl, Phenyl, Biphenylyl, Phenoxyphenyl, Phenylthiophenyl, Phenyl$(C_1-C_4)$-alkyl, Naphthyl, Biphenylyl$(C_1-C_4)$-alkyl, Phenylthiophenyl$(C_1-C_4)$-alkyl, Phenoxyphenyl-$(C_1-C_4)$alkyl, Naphthyl$(C_1-C_4)$-alkyl, Benzothiazol-2-yl, Benzimidazol-2-yl, N-$(C_1-C_4)$-Alkylbenzimidazol-2-yl, Benzyl, Pyridyl, Pyrid-2-yl-methyl, Pyrid-3-yl-methyl, Pyrid-4-yl-methyl, Pyrimidin-2-yl, Pyrimidin-2-yl-methyl, Pyrimidin-4-yl-methyl, Pyrimidin-5-yl-methyl, Furfuryl, Thien-2-yl, Thien-3-yl, Thien-2-yl-methyl, Thien-3-yl-methyl, Isoxazol-4-yl-methyl, Thiazol-2-yl-methyl, Thiazol-5-yl-methyl, Thiazol-5-yl-eth-2-yl, $(C_2-C_3)$-Alkyl-X-aryl (mit X wie vorstehend definiert und Aryl gleich Phenyl, Benzyl, Thien-2-yl-methyl, Thien-3-yl-methyl), Benzothiazol-2-yl-methyl, Chinolinyl-methyl, Pyridyl-phenyl-methyl,

wobei die genannten Ringsysteme unsubstituiert oder durch 1, 2 oder 3 Substituenten, die gleich oder verschieden sind und jeweils F, Cl, Br, J, $CF_3$, $(C_1-C_4)$-Alkyl, $(C_1-C_4)$-Alkoxy bedeuten, substituiert sind; die Ringsysteme Pyrimidinyl und Thienyl jedoch auch durch einen Phenylrest, der unsubstituiert oder seinerseits wie vorstehend angegeben, substituiert ist,

3) 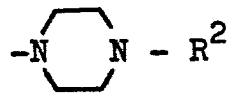

mit $R^2$= $(C_1-C_8)$-Alkyl, $(C_5-C_8)$-Cycloalkyl, Acetyl, Phenyl, Benzyl; wobei der Phenylkern jeweils unsubstituiert oder mit 1-3 F, Cl, Br, J, $CF_3$, $(C_1-C_4)$-Alkyl- oder $(C_1-C_4)$-Alkoxygruppen substituiert ist

4) 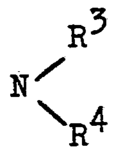 mit $R^3$ und $R^4$ gleich oder verschieden $(C_1-C_4)$Alkyl

5. 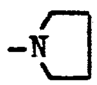 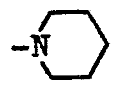 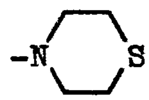 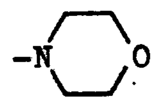

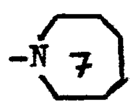 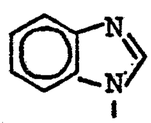 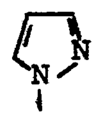

wobei mindestens eine der beiden Gruppen Y die Bedeutung von Y 1) hat;

Z = OH, $(C_1-C_4)$-Alkylcarbonyloxy, F, Cl, Br, $(C_1-C_4)$-Alkoxy, Benzyloxy, unsubstituiert oder 1-2fach substituiert mit F, Cl, Br, $CF_3$;

und ihre Salze mit physiologisch verträglichen Säuren

mit Ausnahme derjenigen Verbindungen, in denen gleichzeitig

a) beide Y die Bedeutung von Y 1) mit W=N und
b) A die Bedeutung Phenyl, gegebenenfalls substituiert mit 1-3 Substituenten, die unabhängig voneinander ausgewählt sind aus F, Cl, Br, J, $CF_3$, $(C_1-C_4)$-Alkyl und $(C_1-C_4)$-Alkoxy, oder 5-Chlorpyrid-2-yl und
c) Z die Bedeutung wie oben angegeben

haben.

Bevorzugt sind Verbindungen I, in denen mindestens einer der Substituenten folgende Bedeutung hat:

A: Phenyl, Biphenyl, 1,2,3,4-Tetrahydronaphthyl, Thienyl, Indanyl, jeweils unsubstituiert oder im aromatischen Ring mit ein oder zwei Substituenten, die gleich oder verschieden sind und jeweils F, Cl, Br, $CF_3$, $(C_1-C_4)$-Alkyl oder $(C_1-C_4)$-Alkoxy bedeuten, substituiert sind

Y: 1) 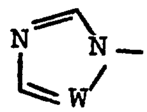 mit W= CH oder N

2) $-X-R^1$ mit

$X = O, S$

$R^1$ = $(C_1-C_{15})$-Alkyl, geradkettig oder verzweigt, $(C_2-C_{15})$-Alkenyl, geradkettig oder verzweigt, ein- oder mehrfach ungesättigt, Phenyl, Biphenylyl, Phenoxyphenyl, Phenylthiophenyl, Phenyl-$(C_1-C_2)$alkyl, Naphthyl, Biphenylyl$(C_1-C_2)$alkyl, Naphthyl$(C_1-C_2)$alkyl, Benzothiazol-2-yl, Benzimidazol-2-yl, Furfuryl, Thien-2-yl-methyl, Thieny-3-yl-methyl, Isoxazol-4-yl-methyl, Pyrid-3-yl-methyl, Pyrid-4-yl-methyl, Ethyl-thio-aryl mit Aryl gleich Phenyl, Benzyl, Thien-2-yl-methyl, Thien-3-yl-methyl, wobei die genannten Ringsysteme unsubstituiert oder durch 1, 2 oder 3 Substituenten, die gleich oder verschieden sind und jeweils F, Cl, Br, $CF_3$, Methyl, Methoxy-Gruppen bedeuten, substituiert sind,

3.) 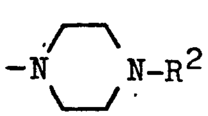 mit

$R^2$= Phenyl, Benzyl, jeweils unsubstituiert oder mit 1 oder 2 F, Cl, Br, $CF_3$, Methyl oder Methoxy-Gruppen substituiert,

Z = OH, $OCH_3$, F, Cl

wobei mindestens eine der beiden Gruppen Y die Bedeutung von Y 1) hat,

und ihre Salze mit physiologisch verträglichen Säuren.

Ganz besonders bevorzugt sind Verbindungen I, bei denen mindestens einer der Substituenten folgende Bedeutung hat:

A Phenyl, Thienyl, jeweils unsubstituiert oder substituiert durch 1 oder 2 F- oder Cl-Atome, Methyl, Methoxy

Y 1) 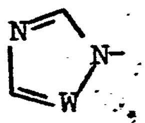 mit W = CH oder N

2) $-X-R^1$ mit

X = O, S

$R^1$= $(C_1-C_{12})$-Alkyl (geradkettig oder verzweigt), Geranyl, Neryl, Phenyl, Benzyl, Naphthyl, Thien-2-yl-methyl Thien-3-yl-methyl, Isoxazol-4-yl-methyl, Pyrid-3-yl-methyl, Pyrid-4-yl-methyl, jeweils unsubstituiert oder 1- oder 2-fach substituiert durch F, Cl, Methyl, Methoxy

Z = OH

wobei mindestens eine der beiden Gruppen Y die Bedeutung von Y 1) hat,

und ihre Salze mit physiologisch verträglichen Säuren.

Die als Substituenten oder in Zusammenhang mit anderen Substituenten auftretenden $(C_1-C_{12})$-, bzw. $(C_1-C_4)$-Alkylgruppen können unverzweigt oder verzweigt sein und beispielsweise die Methyl-, Ethyl-, Propyl-, 1-Methylethyl-, Butyl-, 2-Methylpropyl-, 1,1-Dimethyl-ethyl-, Pentyl-, Hexyl-, Heptyl-Oktyl-, Nonyl-, Decyl-, Undecyl-, Dodecylgruppe bedeuten; die durch Fluor oder Chlor substituierten Alkylgruppen

können beispielsweise die Trifluormethyl-, Trichlormethyl, 1,1,2,2-Tetrafluorethyl oder die Nonafluorbutylgruppe bedeuten; die ($C_3$-$C_8$)-Cycloalkylgruppen können die Cyclopropyl-, Cyclobutyl, Cyclopentyl-, Cyclohexyl-, Cycloheptyl- oder Cyclooctyl-Gruppe bedeuten; die ($C_3$-$C_8$)-Cycloalkyl-($C_1$-$C_4$)alkylgruppen können beispielsweise die Cyclopropylmethyl-, Cyclopentylmethyl-, Cyclohexylmethyl-, Cyclohexylethyl-, Cycloheptylmethyl- oder Cyclooctylmethyl-Gruppe bedeuten.

Die vorliegende Erfindung betrifft die Verbindungen (I) in Form der freien Base oder eines Säureadditionssalzes oder eines physiologisch hydrolysierbaren und akzeptablen Derivates.

Beispiele pharmazeutisch akzeptabler salzbildender Säuren sind anorganische Säuren wie Salzsäure, Bromwasserstoffsäure, Jodwasserstoffsäure, Schwefelsäure, Phosphorsäure, oder Salpetersäure oder organische Säuren wie Malonsäure, Oxalsäure, Gluconsäure, Camphersulfonsäure, Benzolsulfonsäure, Essigsäure, Propionsäure oder p-Toluolsulfonsäure.

0237917

Als physiologisch hydrolysierbare und akzeptable Derivate sind beispielsweise an der Hydroxygruppe veresterte Derivate geeignet, die unter physiologischen Bedingungen zu den freien Säuren hydrolysierbar sind, die ihrerseits wieder physiologisch akzeptierbar sind, d.h. bei den notwendigen Dosen nicht toxisch sind.

Die Verbindungen (I) weisen mindestens ein asymmetrisches C-Atom auf und können daher als Enantiomere und Diastereomere auftreten. Die Erfindung umfaßt sowohl die reinen Isomeren als auch deren Gemische. Die Gemische von Diastereomeren können nach gebräuchlichen Methoden, zum Beispiel durch selektive Kristallisation aus geeigneten Lösungsmitteln oder durch Chromatographie an Kieselgel oder Aluminiumoxid in die Komponenten aufgetrennt werden. Racemate können nach üblichen Methoden in die Enantiomeren aufgetrennt werden, so zum Beispiel durch Salzbildung mit einer optisch aktiven Säure, Trennung der diastereomeren Salze und Freisetzung der reinen Enantiomeren mittels einer Base.

Die Erfindung betrifft weiterhin ein Verfahren zur Herstellung von Verbindungen der Formel (I), dadurch gekennzeichnet, daß man eine Verbindung der Formel (II)

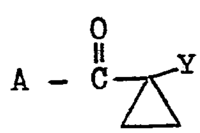

II,

in welcher A und Y die in Anspruch 1 genannten Bedeutungen haben, mit einem Schwefelylid der Formel III,

$$(H_3C)_2\underset{(O)_p}{S}CH_2 \qquad III,$$

worin p Null oder 1 bedeutet,

zu einer Verbindung der Formel IV

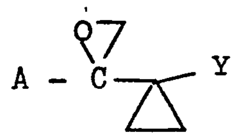

IV

umsetzt,
und anschließend die Verbindung IV mit einem Nucleophil der Formel Y-M umsetzt, in welcher
Y die im Anspruch 1 genannten Bedeutungen hat und
M Wasserstoff oder ein Metalläquivalent ist,
wobei einer Verbindung I mit Z= OH entsteht, und
falls gewünscht, diese Verbindung I acyliert oder alkyliert und gegebenenfalls am Schwefel einer Thioethergruppe zum Sulfoxid oder Sulfon oxidiert
und gegebenenfalls die Verbindung I mit Z= OH in eine Verbindung mit Z= F, Cl oder Brom überführt und eine so erhaltene Verbindung der Formel (I) in Form der freien Base oder eines Säureadditionssalzes isoliert.

Zur Herstellung der Verbindungen der Formel (I) wird in einem ersten Reaktionsschritt zur Herstellung der Verbindungen der Formel (IV) ein Keton der Formel (II), in der A und Y die oben angegebenen Bedeutungen haben, mit einem Schwefelylid der Formel (III) in einem inerten Lösungsmittel, vorzugsweise Dimethylsulfoxid, oder in Gemischen von Dimethylsulfoxid mit anderen inerten Lösungsmitteln, z.B. Tetrahydrofuran, umgesetzt. Dabei ist bei Verwendung eines Schwefelylids der Formel (III), für das p = 0 ist, ein Temperaturbereich zwischen -10° und 50°C, vorzugsweise zwischen 0° und 30°C zweckmäßig; bei Verwendung eines Schwefelylids der Formel (III), für das p = 1 ist, ein Temperaturbereich zwischen 0° und 80°, vorzugsweise zwischen 20° und 60°.

Die Umwandlung der Zwischenprodukte der Formel IV in die Endprodukte der Formel I mit Z= OH erfolgt in einem zweiten Reaktionsschritt durch Umsetzung mit einer Verbindung der Formel Y-M, in der Y und M die oben angegebenen Bedeutungen haben.

Die vorgenannte Umsetzung erfolgt in einem Temperaturbereich von 20-160°C, gegebenenfalls in einem inerten Lösungsmittel, gegebenenfalls in Anwesenheit einer Base.

Geeignete Lösungsmittel sind beispielsweise N,N-Dimethylformamid, N,N-Dimethylacetamid, Dimethylsulfoxid, Sulfolan, N-Methylpyrrolidon-2, Dioxan, Tetrahydrofuran, Acetonitril, 4-Methyl-2-pentanon, Methanol, Ethanol, Isopropylalkohol, Propanol, Butanol, Pentanol, Tert.-butylalkohol, Methylglykol, Eisessig, Methylenchlorid oder ein aromatischer Kohlenwasserstoff wie Benzol, Chlorbenzol, Nitrobenzol, Toluol oder Xylol oder Wasser. Es können auch Gemische der beispielhaft genannten Lösungsmittel verwendet werden.

Geeignete Basen sind beispielsweise Alkalimetall- oder Erdalkali-carbonate, hydrogencarbonate, -hydroxide, -alkoholate oder -hydride wie z.B. Natriumcarbonat, Natriumhydrogencarbonat, Kaliumcarbonat, Natriumhydroxid, Natriummethylat oder Natriumhydrid, oder organische Basen z.B. tertiäre Amine wie Triethylamin, Tributylamin, Ethylmorpholin oder Pyridin, Dimethylaminopyridin, Chinolin oder 1,5-Diazabicyclo[5,4,0]undec-5-en(1,8,7) (DBU), 1-H-Imidazol oder 1-H-1,2,4-Triazol.

Die Umsetzung der Verbindungen der Formel II mit den Verbindungen der Formel III zu den Zwischenprodukten der Formel IV und Umsetzung dieser mit solchen der Formel Y-M zu Verbindungen der Formel I mit Z= OH kann auch im Eintopfverfahren durchgeführt werden.

Hierzu stellt man zunächst, wie oben angegeben, durch Umsetzung einer Verbindung der Formel II mit einer Verbindung der Formel III eine Lösung einer Verbindung der Formel IV in einem der oben angegebenen inerten Lösungsmittel her. Anschließend gibt man zu dieser Lösung eine mindestens äquivalente Menge einer Verbindung der Formel Y-M und gegebenenfalls eine katalytische oder äquivalente Menge eine der oben genannten Basen und verfährt weiter, wie oben angegeben.

Verbindungen der Formel I, in denen Z $(C_1-C_4)$-Alkoxy, Benzyloxy unsubstituiert oder ein oder zweifach mit F, Cl, Br oder $CF_3$ substituiert bedeutet, werden hergestellt, indem man eine Verbindung der Formel I mit Z= OH mit einem entsprechenden Alkyl- oder Benzylhalogenid, vorzugsweise einem Chlorid oder Bromid umsetzt, in Anwesenheit einer Base, in einem inerten Lösungsmittel, in einem Temperaturbereich von 0 - 100°C. Als Lösungsmittel und Basen werden vorzugsweise diejenigen verwendet, die zur Umsetzung von Verbindungen der Formel IV mit Y-M angegeben sind.

Verbindungen der Formel I, in denen Z $(C_1-C_4)$-Alkylcarbonyloxy bedeutet, werden hergest ellt, indem man eine Verbindung der Formel I mit Z= OH mit einem entsprechenden Alkylcarbonsäurehalogenid, vorzugsweise einem Alkylcarbonsäurechlorid oder Alkylcarbonsäureanhydrid umsetzt, in Gegenwart einer Base, in einem inerten Lösungsmittel, in einem Temperaturbereich von -10 bis 120°C.

Als Lösungsmittel und Basen werden vorzugsweise diejenigen verwendet, die zur Umsetzung von Verbindungen der Formel IV mit Y-M angegeben sind.

Verbindungen der Formel I, in denen Z F, Cl oder Brom bedeutet, werden hergestellt, in dem man eine Verbindung der Formel I mit Z= OH mit Thionylchlorid, Thionylbromid, Schwefeltetrafluorid oder Diethylaminoschwefeltrifluorid $(Et_2NSF_3)$ umsetzt, in einem inerten Lösungsmittel, ge-

0237917

gebenenfalls in Gegenwart einer Base, in einem Temperaturbereich von 0 - 80°C.

Als Lösungsmittel und Basen werden vorzugsweise diejenigen verwendet, die zur Umsetzung von Verbindungen der Formel IV mit Y-M angegeben sind.

Verbindungen der Formel I, die eine Thioethergruppe enthalten, können am Schwefel zu einem Sulfoxid oder Sulfon oxidiert werden. Dazu werden solche Verbindungen der Formel I mit einem Oxidationsmittel wie z.B. Wasserstoffperoxid oder m-Chlorperbenzoesäure umgesetzt, in einem inerten Lösungsmittel, in einem Temperaturbereich von -10 bis 80°C. Zur Herstellung von Sulfoxiden setzt man dazu ein Moläquivalent Oxidationsmittel ein, zur Herstellung von Sulfonen zwei Moläquivalente, gegebenenfalls auch einen Überschuß.

Als Lösungsmittel werden vorzugsweise diejenigen verwendet, die zur Umsetzung von Verbindungen der Formel IV mit Y-M angegeben sind.

Herstellung der Ausgangsstoffe

Zur Herstellung von Verbindungen der Formel II

Verbindungen der Formel II in denen A und Y die in Anspruch 1 angegebenen Bedeutungen haben, werden vorteilhaft hergestellt, indem man eine Verbindung der Formel VI

$$A-\overset{\overset{\displaystyle O}{\|}}{C}-\underset{\underset{\displaystyle Br}{|}}{CH}-CH_2-CH_2-Cl \qquad VI$$

in der A die in Anspruch 1 angegebenen Bedeutungen hat, mit einer Verbindung der Formel Y-M umsetzt, in der Y und M die in Anspruch 1 angegebenen Bedeutungen haben.

Die vorstehende Umsetzung erfolgt entweder

a) einstufig, in einem inerten Lösungsmittel, in Anwesenheit von mindestens 2 Moläquivalenten einer Base, in einem Temperaturbereich von 0 - 120°C, oder

b) zweistufig, indem man in einem inerten Lösungsmittel, in Anwesenheit von mindestens 1 Moläquivalent einer Base, in einem Temperaturbereich von 0 - 50°C zunächst eine Verbindung der Formel VII herstellt,

$$A-\overset{\overset{O}{\|}}{C}-\underset{\underset{Y}{|}}{CH}-CH_2-CH_2-Cl \qquad VII$$

in der A und Y die oben genannten Bedeutungen haben.

Als Lösungsmittel und Basen werden wie auch bei der einstufigen Variante a) vorzugsweise diejenigen verwendet, die zur Umsetzung von Verbindungen der Formel IV mit Y-M angegeben sind.

In einem zweiten Reaktionsschritt werden die Verbindungen der Formel VII, nach gegebenenfalls erfolgter Aufarbeitung und Reinigung, unter den Bedingungen einer Phasentransferreaktion zu Verbindungen der Formel II umgesetzt. Dazu wird eine Lösung der Verbindungen der Formel VII in einem inerten Lösungsmittel unter kräftigem Rühren, in Anwesenheit eines Phasentransferkatalysators mit einer Base umgesetzt, vorzugsweise einem gepulvertem Alkalihydroxid oder -carbonat, wie z.B. Natrium- oder Kaliumhydroxid oder Carbonat oder einer konzentrierten wäßrigen Lösung derselben, vorzugsweise in einem Temperaturbereich von 20 - 100°C.

Als Lösungsmittel werden diejenigen verwendet, die zur Umsetzung von Verbindungen der Formel IV mit Y-M angegeben sind.

Geeignete Phasentransferkatalysatoren sind quaternäre Ammonium- oder Phosphoniumsalze sowie Kronenether oder Polyethylenglykole. Beispiele solcher Salze finden sich

zusammengestellt in der Monographie von W.P. Weber und G.W. Gockel, Phase Transfer Catalysis in Organic Synthesis, Berlin, Springer Verlag, 1977. Als besonders geeignet für die vorliegenden Zwecke erwiesen sich Tetrabutylammoniumbromide und Benzyltriethylammoniumchlorid.

Das zweistufige Verfahren ist besonders dann vorteilhaft, wenn Verbindungen der Formel II hergestellt werden, in denen A oder Y einen leicht alkylierbaren Rest enthalten.

Die Verbindungen der Formel II können auch hergestellt werden, indem man eine Verbindung der Formel VIII,

$$A - \overset{\overset{O}{\|}}{C} - CH_2 - Y \qquad \text{VIII}$$

in der A und Y die oben angegebenen Bedeutungen haben, mit mindestens einem Moläquivalent 1,2-Dibromethan umsetzt.

Die vorstehende Umsetzung erfolgt vorzugsweise in einem Temperaturbereich von 20 - 100°C unter den Bedingungen einer Phasentransferreaktion wie oben angegeben.

Die hier als Ausgangsmaterial verwendeten Verbindungen der Formeln II und VII, deren Herstellung in der vorliegenden Anmeldung beschrieben ist, sind ebenfalls beschrieben in dem parallelen deutschen Patent ........(P 36 08 727.0, HOE 86/F 059).

Verbindungen der Formel VI können aus den entsprechenden ω-Chlorketonen durch Umsetzung mit $Br_2$ gegebenenfalls in einem Lösungsmittel, vorzugsweise in Eisessig oder Methylenchlorid in einem Temperaturbereich von 0 - 60°C hergestellt werden. Die benötigten ω-Chlorketone sind bekannt oder können analog dazu hergestellt werden. Die Ausgangsprodukte der Formel III, Y-M und VIII sind ebenfalls bekannt oder können nach bekannten Verfahren erhalten werden.

Die Erfindung betrifft weiterhin ein Verfahren zur Herstellung der Verbindungen der Formel I, nach welchem man eine Verbindung der Formel II

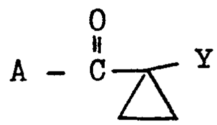

II

in welcher A und Y die in Anspruch 1 genannten Bedeutungen haben mit einem Phosphorylid zu einer Verbindung der Formel V

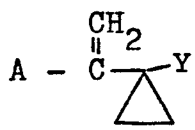

V

umsetzt,
und anschließend die Verbindung V mit einem organischen Peroxid zu einer Verbindung der Formel IV

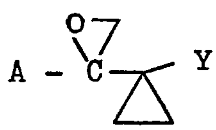

IV

umsetzt,
und anschließend die Verbindung IV mit einem Nucleophil der Formel Y-M umsetzt, in welcher
Y die in Anspruch 1 genannten Bedeutungen hat und
M Wasserstoff oder ein Metalläquivalent ist,
wobei eine Verbindung I mit Z = OH entsteht und, falls gewünscht, diese Verbindung I acyliert, alkyliert oder in eine Verbindung mit Z= F, Cl oder Br, überführt, und gegebenenfalls am Schwefel einer Thioethergruppe zum Sulfoxid oder Sulfon oxidiert,
und gegebenenfalls in das Salz mit einer physiologisch verträglichen Säure überführt.

Zur Herstellung der Verbindungen der Formel I wird in einem ersten Reaktionsschritt zur Herstellung von Verbindungen der Formel V ein Keton der Formel II, in der A und

Y die oben angegebenen Bedeutungen haben, mit einem Phosphorylid in einem inerten Lösungsmittel, in einem Temperaturbereich von -60 bis +80°C umgesetzt.

Das dabei verwendete Phosphorylid wird durch Umsetzung eines Methyltriarylphosphoniumsalzes, vorzugsweise Methyltriphenylphosphonium-Chlorid, Bromid oder Jodid, mit einer starken Base in einem inerten Lösungsmittel hergestellt. Geeignete Basen sind z.B. Alkali- oder Erdalkalimetallhydride-, -amide oder -alkoholate, Natriumbistrimethylsilylamid oder Lithiumorganyle wie z.B. n-Butyl- oder Phenyl-Lithium.

Als Lösungsmittel werden diejenigen verwendet, die zur Umsetzung von Verbindungen der Formel IV mit Y-M angegeben sind.

In einem zweiten Reaktionsschritt zur Herstellung von Verbindungen der Formel IV wird eine Verbindung der Formel V mit einem Oxidationsmittel umgesetzt, in einem inerten Lösungsmittel, in einem Temperaturbereich von -10 bis 80°C, gegebenenfalls in Anwesenheit einer Base, einer Base und eines Nitrils wie z.B. Benzonitril, oder Thionylbis-imidazol oder -triazol.

Geeignete Oxidationsmittel sind z.B. $H_2O_2$ oder Percarbonsäuren wie z.B. Peressigsäure, Trifluorperessigsäure, Perbenzoesäure oder m-Chlorperbenzoesäure.

Als Basen und Lösungsmittel werden diejenigen verwendet, die zur Umsetzung von Verbindungen der Formel IV mit Y-M angegebenen sind.

In einem dritten Reaktionsschritt zur Herstellung von Verbindungen der Formel I mit Z= OH aus Verbindungen der Formel IV verfährt man wie im vorstehenden Verfahren angegeben.

Diese Verbindungen der Formel I mit Z= OH können, falls gewünscht, acyliert, alkyliert oder in eine Verbindung mit Z= F, Cl oder Br überführt, und gegebenenfalls am Schwefel einer Thioethergruppe zum Sulfoxid oder Sulfon oxidiert, und gegebenenfalls in das Salz mit einer physiologisch verträglichen Säure überführt werden, wie im vorstehenden Verfahren angegeben.

Die Verbindungen der Formel I, ihre Säureadditionssalze und ihre physiologisch hydrolysierbaren Derivate sind wertvolle Arzneimittel. Sie wirken insbesondere antimikrobiell und eignen sich zur Vorbeugung und Behandlung von Pilzinfektionen beim Menschen und bei verschiedenen Säugetierarten.

Die neuen Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als Indikationsgebiete in der Humanmedizin können beispielsweise genannt werden:

Dermatomykosen und Systemmykosen durch Trichophyton mentagrophytes und andere Trichophytonarten, Mikrosporonarten,

Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Zur vorliegenden Erfindung gehören phamazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa mindestens 0,05, vorzugsweise 0,1, insbesondere 0,5 bis etwa höchstens 200, vorzugsweise 100, insbesondere 10 mg/kg Körpergewicht je 24 Stunden, gegebenenfalls in Form mehrerer Einzelgaben zur Erzielung der gewünschten Ergebnisse zu verabreichen, bezogen auf einen Erwachsenen eines Gewichts von 75 kg. Die Gesamtmenge wird in 1 bis 8, vorzugsweise in 1 bis 3 Einzelsdosen verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung

der jeweils erforderlichen optimalen Dosierung und Applikationsart der Wirkstoffe kann durch jeden Fachmann aufgrund seines Fachwissens leicht erfolgen.

Die neuen Verbindungen der allgemeinen Formel (I) sind auch als Biozide wirksam, vorzugsweise die Triazolverbindungen, in denen W Stickstoff bedeutet. Sie zeichnen sich insbesondere durch ihre fungizide Wirksamkeit bei phytopathogenen Pilzen aus. Selbst bereits in das pflanzliche Gewebe eingedrungene pilzliche Krankheitserreger lassen sich erfolgreich bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der neuen Verbindungen erfaßt eine Vielzahl verschiedener phytopathogener Pilze, wie z.B. Piricularia oryzae, Plasmopara viticola, verschiedene Rostarten, vor allem aber Venturia inaequalis, Cercospora beticola und echte Mehltaupilze im Obst-, Gemüse-, Getreide- und Zierpflanzenbau.

Die neuen Verbindungen der allgemeinen Formel (I) eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die neuen Verbindungen können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate

und Dispergiermittel z.B. ligninsulfonsaures Natrium, 2,2'-dinapthylmethan-6,6'-di-sulfonsaures Natrium, dibutylnaphthalin-sulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze, wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinit oder von granuliertem Inertmaterial. Auch können geeignete Wirkstoffe in der für die Herstellung von Düngemittelgranulaten

üblichen Weise - gewünschtenfalls in Mischung mit Düngemitteln, granuliert werden.

In Spritzpulvern beträgt die Wirkstoffkonzentration z.B. etwa 10-90 Gew.-%, der Rest zu 100 Gew.-% besteht aus üblichen Formulierungsbestandteilen. Bei emulgierbaren Konzentraten kann die Wirkstoffkonzentration etwa 10-80 Gew.-% an Wirkstoff, bei versprühbaren Lösungen etwa 1-20 Gew.-% betragen. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z.B. Insektiziden, Akariziden, Herbiziden, Düngemitteln, Wachstumsregulatoren oder weiteren Fungiziden sind gegebenenfalls möglich, wobei u.U. auch synergistische Wirkungssteigerungen erzielt werden können.

Im folgenden seien einige Formulierungsbeispiele angeführt:

Ein Stäubemittel wird erhalten, indem man 10 Gewichtsteile Wirkstoff und 90 Gewichtsteile Talkum als Inertstoff mischt und in einer Schlagmühle zerkleinert.

Ein in Wasser leicht dispegierbares, benetzbares Pulver wird erhalten, indem man 25 Gewichtsteile Wirkstoff, 65 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat stellt man her, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z.B. ca. 255 bis über 377°C) mischt und in einer Reibkugelmühle auf eine Feinheit von unter 5 Mikron vermahlt.

Ein emulgierbaren Konzentrat läßt sich herstellen aus 15 Gewichtsteilen Wirkstoff, 75 Gewichtsteilen Cyclohexanon als Lösungsmittel und 10 Gewichtsteilen oxethyliertem Nonylphenol (10 AeO) als Emulgator.

Die nachfolgenden Beispiele dienen zur näheren Erläuterung der Erfindung, ohne dieselbe einzuschränken.

<u>Beispiel 1</u>

1-[1-(4-Chlorphenyl)-1-hydroxy-2-(1,2,4-triazol-1-yl)-ethyl]-1-octylthio-cyclopropan

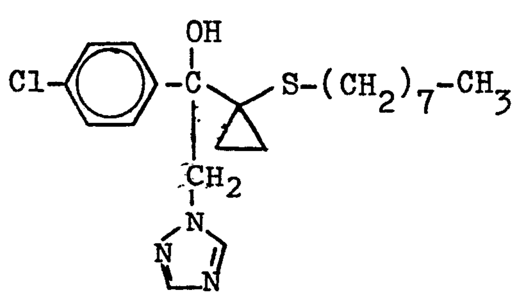

Zur Suspension aus 1,65 g = 55 mmol Natriumhydrid (80 %ige Suspension in Öl) in 70 ml trockenem Dimethylsulfoxid wurden portionsweise unter Kühlen und Rühren bei 20°C 12,1 g 55 mmol Trimethylsulfoxoniumjodid eingetragen und bis zum Ende der Wasserstoffentwicklung nachgerührt. Anschließend tropfte man die Lösung von 16,2 g = 50 mmol 1-(4-Chlorbenzoyl)-1-octylthio-cyclopropan in 20 ml trockenem Dimethylsulfoxid schnell zu, rührte eine Stunde bei Zimmertemperatur und eine Stunde bei 40°C. Anschließend gab man 5,2 g = 75 mmol 1,2,4-Triazol und danach 2,3 g = 25 mmol 1,2,4-Triazol-Natriumsalz portionsweise zu und rührte 2 Stunden bei 50°C und 6 Stunden bei 80°C. Zur Aufarbeitung wurde die Lösung in die dreifache Menge Eiswasser gegossen, mit Methylenchlorid extrahiert, die organische Phase zweimal mit 50 ml 2n NaOH gewaschen, mit Natriumsulfat getrocknet und im Wasserstrahlvakuum eingeengt. Das verbleibende Öl löste man in 20 ml Methanol, worauf die Substanz kristallisierte. Man erhielt 12,2 g (60 % d.Th.) vom Schmp. 117 - 118°C (aus Methanol).

Herstellung der Ausgangsprodukte

1-(4-Chlorbenzoyl)-1-octylthio-cyclopropan

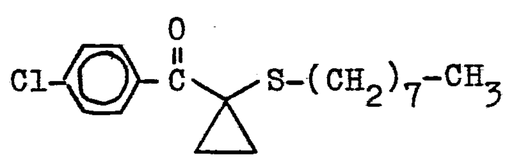

Zur Lösung von 8,8 g = 60 mmol Octylmercaptan und 7 g = 70 mmol Triethylamin in 60 ml Aceton tropfte man unter Stickstoffatmosphäre, Rühren und Kühlen bei 0°C die Lösung von 17,8 g = 60 mmol 1-Brom-3-chlor-propyl-(4-chlorphenyl)-keton in 40 ml Aceton, ließ auf Zimmertemperatur aufwärmen und rührte 15 Stunden. Der entstandene Niederschlag wurde abgesaugt, mit Aceton gewaschen und verworfen. Die organische Phase wurde im Wasserstrahlvakuum eingeengt, der

ölige Rückstand (Verbindung der Formel VII) in 50 ml Methylenchlorid aufgenommen, mit 20 ml 50 %ger Natronlauge und 0,4 g Tetrabutylammoniumbromid versetzt und 8 Stunden bei intensiver Rührung zum Rückfluß gekocht. In die kalte Mischung gab man unter Rühren 100 ml Wasser und arbeitete auf wie vorstehend beschrieben. Der erhaltene Rückstand (19 g) wurde im Ölpumpenvakuum destilliert. Man erhielt 16,1 g (82 % d.Th.) vom Siedepunkt 165 - 170°C bei 0,1 Torr.

1-Brom-3-chlor-propyl-(4-chlorphenyl)-keton

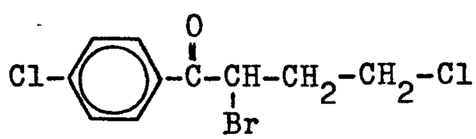

Zur Lösung von 262 g = 1,2 mol 3-Chlorpropyl-(4-chlorphenyl)-keton in 480 ml Eisessig tropfte man unter Rühren und Kühlen bei 30°C 192 g = 1,2 mol Brom, rührte 4 Stunden bei Zimmertemperatur, saugte den Niederschlag ab, wusch mit kaltem Diisopropylether nach, trocknete und erhielt 335 g (94 % d.Th.) vom Schmp.: 73 - 74°C.
Gemäß dem vorstehenden Beispiel wurden die übrigen als Ausgangsprodukte verwendeten 1-Brom-3-chlor-propyl-ketone hergestellt.

<u>Beispiel 2</u>

1-[1-(4-Chlorphenyl)-1-hydroxy-2-(imidazol-1-yl)-ethyl]-1-(4-chlorphenoxy)-cyclopropan

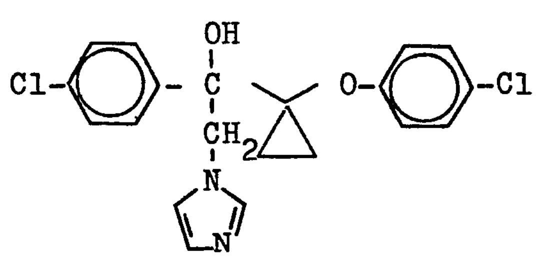

a) 2-(4-Chlorphenyl)-2-[1-(4-chlorphenoxy)-cyclopropan-1-yl]-oxiran (Verbindung der Formel IV).

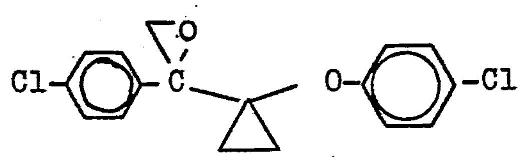

Zunächst setzte man in trockenem Dimethylsulfoxid 55 mmol Natriumhydrid, 55 mmol Trimethylsulfoxoniumjodid und 15,4 g= 50 mmol 1-(4-Chlorbenzoyl)-1-(4-chlorphenoxy)-cyclopropan miteinander um und arbeitete auf, wie im Beispiel 1 angegeben. Nach dem Verreiben des öligen Rückstandes mit Ether kristallisierten 11,2 g (70 % d.Th.) vom Schmp.: 97°C aus.

b) 1-[1-(4-Chlorphenyl)-1-hydroxy-2-(imidazol-1-yl)-ethyl]-1-(4-chlorphenoxy)-cyclopropan

11,2 g = 35,5 mmol des vorstehenden Oxirans wurden mit 7,2 g = 106,5 mmol Imidazol in Substanz unter Rühren auf 110°C erhitzt. Bei dieser Temperatur setzte eine exotherme Reaktion bis ca. 130°C ein. Man entfernte die Heizquelle bis die Innentemperatur wieder 110°C erreichte, rührte anschließend 2 Stunden bei 120°C, goß die heiße Mischung in die dreifache Menge Eiswasser und arbeitete auf, wie in Beispiel 1 angegeben. Nach dem Verreiben des harzigen Rückstandes mit Ether kristallisierten 11 g (80 % d.Th.) vom Schmp.: 172 - 173°C.

Herstellung des Ausgangsproduktes
1-(4-Chlorbenzoyl)-1-(4-chlorphenoxy)-cyclopropan

Zur Suspension von 17,2 g = 125 mmol gepulvertem Kaliumcarbonat und 6,4 g = 50 mmol p-Chlorphenol in 60 ml Acetonitril, tropfte man unter Rühren bei Raumtemperatur die Lösung von 14,8 g = 50 mmol 1-Brom-3-chlorpropyl-(4-chlorphenyl)-keton in 30 ml Acetonitril. Man rührte 5 Stunden bei Raumtemperatur, gab 0,4 g Tetrabutylammoniumbromid zu, rührte 10 Stunden am Rückfluß, arbeitete auf, wie im Beispiel 1 angegeben und erhielt 12,6 g (82 % d.Th.) vom Schmp.: 69 - 71°C. (aus Methanol)

Beispiel 3

1-[1-(4-Methylphenyl)-1-hydroxy-2-(4-chlorphenylthio)-ethyl]-1-(imidazol-1-yl)-cyclopropan

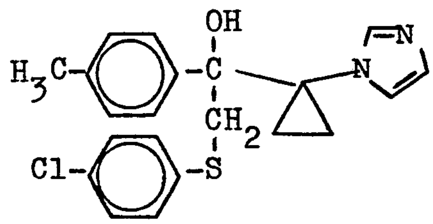

Zunächst setzte man in trockenem Dimethylsulfoxid 55 mmol Natriumhydrid, 55 mmol Trimethylsulfoxoniumjodid und 12,0 g = 50 mmol 1-(4-Methylbenzoyl)-1-(imidazol-1-yl)-cyclopropan miteinander um, wie in Beispiel 1 angegeben und erhielt die Lösung eines entsprechenden Oxirans der Formel IV. Dazu tropfte man unter Stickstoffatmosphäre bei 20°C, die Lösung von 7,2 g = 50 mmol p-Chlorthiophenol in 15 ml trockenem Dimethylsulfoxid, rührte 4 Stunden bei 50°C nach und arbeitete auf, wie in Beispiel 1 angegeben. Nach dem Verreiben des harzigen Rückstandes mit Methanol kristallisieren 12,1 g (63 % d.Th.) vom Schmp.: 158 - 160°C

Herstellung des Ausgangsproduktes
1-(4-Methylbenzoyl)-1-(imidazol-1-yl)-cyclopropan

Zur Lösung von 102 g = 1,5 mol Imidazol in 150 ml Dimethylformamid gab man unter Rühren und Kühlen bei 0 - 5°C 82,6 g = 0,3 mol 1-Brom-3-chlor-propyl-(4-methylphenyl)-keton portionsweise zu und rührte 15 Stunden bei Zimmertemperatur. Zur Aufarbeitung goß man in 1,5 l Eiswasser, extrahierte zweimal mit je 150 ml Methylenchlorid, wusch die organische

Phase zweimal m it je 100 ml 2n NaOH aus, trocknete über Natriumsulfat und filtrierte. Die so bereitete Lösung der entsprechenden Verbindung der Formel VII tropfte man bei intensiver Rührung und Kühlung bei 20°C in die Mischung aus 70 ml 50 %iger Natronlauge und 1,6 g Tetrabutylammoniumbromid, rührte 8 Stunden bei 20°C und arbeitete auf wie in Beispiel 1 angegeben. Der ölige Rückstand (66,5 g 98 % d.Th.) kristallisierte. Eine Probe davon zeigte den Schmp.: 88 - 90°C aus Methyl-tert.-butylether.

Beispiel 4

Analog Beispiel 1, 2 oder 3, jedoch unter Verwendung entsprechender Verbindungen der Formel II und Y-M, können auch die in Tabelle 1 aufgeführten Verbindungen der Formel I mit Z= OH hergestellt werden.
In Spalte 2 (Variante) ist angegeben, ob nach Beispiel 1, 2 oder 3 verfahren wurde.

Tabelle 1

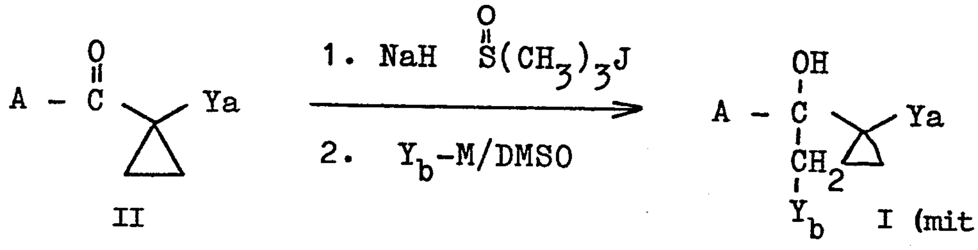


II

I (mit Z= OH)

a) und b) geben die Positionen der Reste Y an;
Im bedeutet 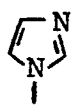, Triaz bedeutet 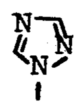

| Beisp.-Nr. | Variante | A | $Y_a$ | $Y_b$ | Schmp. (°C) |
|---|---|---|---|---|---|
| 1.1 | 1 | $-C_6H_4$-4-Cl | $-S-(CH_2)_7-CH_3$ | Im | 99–101 |
| 1.2 | 1 | " | $-S-(CH_2)_3-CH_3$ | Im | 116–118 |
| 1.3 | 1 | " | $-S-(CH_2)_3-CH_3$ | Triaz | 143–144 |
| 1.4 | 1 | $-C_6H_3$-2,4-$Cl_2$ | $-S-(CH_2)_3-CH_3$ | Im | |
| 1.5 | 1 | $-C_6H_4$-4-Cl | 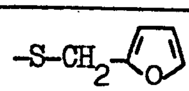 | Triaz | 167–172 |
| 1.6 | 1 | " | " | Im | 161–162 |
| 1.7 | 1 | " | 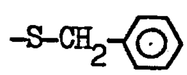 | Triaz | |
| 1.8 | 1 | $-C_6H_4$-4-F | " | Triaz | |
| 1.9 | 1 | $-C_6H_3$-2,4-$F_2$ | " | Triaz | |
| 1.10 | 1 | $-C_6H_4$-4-Cl | 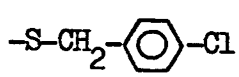 | Im | 185–186 |
| 1.11 | 1 | $-C_6H_4$-4-Cl | " | Triaz | 184–185 |
| 1.12 | 1 | $-C_6H_4$-4-Cl | 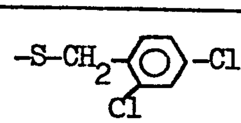 | Im | |

Tabelle 1 Fortsetzung

| Beisp.-Nr. | Variante | A | $Y_a$ | $Y_b$ | Schmp. [°C] |
|---|---|---|---|---|---|
| 1.13 | 1 | $-C_6H_4-4-Cl$ | " | Triaz | 155 |
| 1.14 | 1 | $-C_6H_3-2,4Cl_2$ | 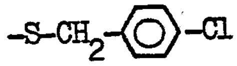 | Im | Öl |
| 1.15 | 1 | $-C_6H_3-2,4-Cl_2$ | 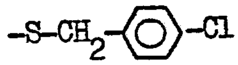 | Triaz | |
| 1.16 | 1 | $-C_6H_3-2,4-F_2$ | " | Triaz | 124–126 |
| 1.17 | 1 | " | " | Im | 174–175 |
| 1.18 | 1 | $-C_6H_4-Cl$ | 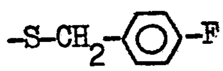 | Triaz | 168–70 |
| 1.19 | 1 | " | 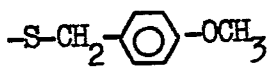 | Triaz | |
| 1.20 | 1 | $-C_6H_4-4-F$ | 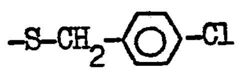 | Im | 170–171 |
| 1.21 | 1 | " | " | Triaz | 168–169 |
| 1.22 | 1 | $-C_6H_4-4-OCH_3$ | " | Im | 157–159 |
| 1.23 | 1 | " | " | Triaz | 171–173 |
| 1.25 | 1 | $-C_6H_4-4-CH_3$ | " | Triaz | |
| 1.26 | 1 | $-C_6H_5$ | " | Triaz | |
| 1.27 | 1 | $-C_6H_4-4-Cl$ | 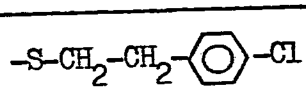 | Triaz | |
| 1.28 | 1 | $-C_6H_4-4-Cl$ | 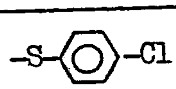 | Im | 164–165 |
| 1.29 | 1 | " | " | Triaz | 128–129 |

Tabelle 1 Fortsetzung

| Beisp.-Nr. | Variante | A | $Y_a$ | $Y_b$ | Schmp. [°C] |
|---|---|---|---|---|---|
| 1.30 | 1 | $-C_6H_4-4-Cl$ | 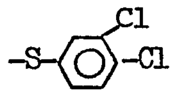 | Im | 152-154 |
| 1.31 | 1 | " | 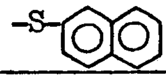 | Im | 189-190 |
| 1.32 | 1 | " | " | Triaz | 185-188 |
| 1.33 | 1 | " | 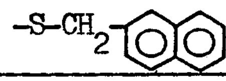 | Triaz | |
| 1.34 | 1 | $-C_6H_4-4-Cl$ | 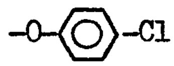 | Triaz | 160-165 |
| 1.35 | 2 | " | 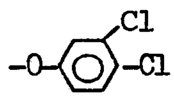 | Im | 170-173 |
| 1.36 | 2 | " | 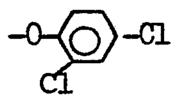 | Im | 209-210 |
| 1.37 | 1 | " | " | Triaz | |
| 1.38 | 2 | " | 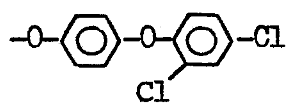 | Im | 180-182 |
| 1.39 | 2 | " | 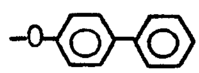 | Im | 168-170 |
| 1.40 | 1 | " | " | Triaz | 135-137 |
| 1.41 | 1 | " | 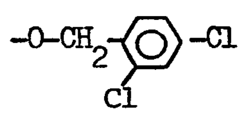 | Triaz | |
| 1.42 | 1 | " | 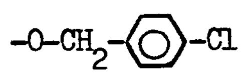 | Triaz | |
| 1.43 | 1 | 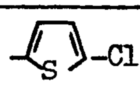 | 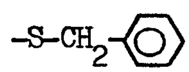 | Triaz | |
| 1.44 | 1 | " | 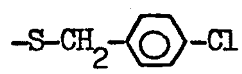 | Triaz | 195-196 |

Tabelle 1 Fortsetzung

| Beisp.-Nr. | Variante | A | $Y_a$ | $Y_b$ | Schmp. [°C] |
|---|---|---|---|---|---|
| 1.45 | 2 | $-C_6H_4-4-Cl$ | 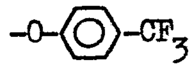 | Im | |
| 1.46 | 1 | $-C_6H_4-4-Cl$ | 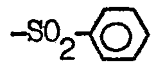 | Im | |
| 1.47 | 1 | 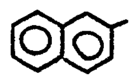 | 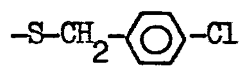 | Triaz | |
| 1.48 | 1 | 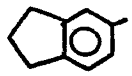 | " | Triaz | |
| 1.49 | 1 | 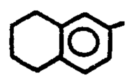 | " | Triaz | |
| 1.50 | 1 | 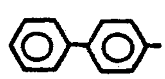 | " | Triaz | |
| 1.51 | 1 | 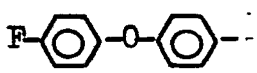 | " | Triaz | |
| 1.52 | 3 | $C_6H_4-4-Cl$ | 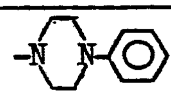 | Im | 196–198 |
| 1.52 | 3 | " | " | Triaz | 135–137 |
| 1.53 | 3 | " | 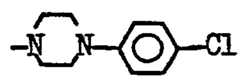 | Triaz | |
| 1.54 | 3 | " | 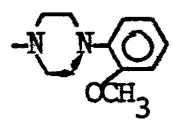 | Triaz | |
| 1.55 | 3 | " | 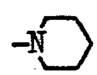 | Triazol | |
| 1.56 | 1 | " | Im | Im | 221–223 |
| 1.57 | 1 | $C_6H_4-4-F$ | Triaz | Im | 191–192 |
| 1.58 | 1 | " | Im | Im | 204–206 |
| 1.59 | 1 | $C_6H_3-2,4-F_2$ | Im | Triaz | 193–194 |

0237917

Tabelle 1 Fortsetzung

| Beisp.-Nr. | Variante | A | $Y_a$ | $Y_b$ | Schmp. [°C] |
|---|---|---|---|---|---|
| 1.60 | 1 | $C_6H_3$-2,4-$F_2$ | Triaz | Im | 205-207 |
| 1.61 | 1 | 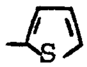 | Triaz | Triaz | 140-141 |
| 1.62 | 1 | 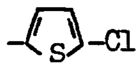 | Triaz | Triaz | 181-183 |
| 1.63 | 1 | 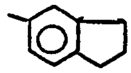 | Triaz | Triaz | |
| 1.65 | 1 | 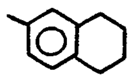 | Triaz | Triaz | |
| 1.66 | 1 | 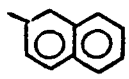 | Triaz | Triaz | |
| 1.67 | 1 | 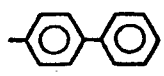 | Triaz | Triaz | |
| 1.68 | 1 | 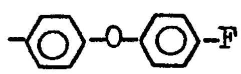 | Triaz | Triaz | |
| 1.69 | 3 | $C_6H_4$-4-Cl | Im | $-S-(CH_2)_7-CH_3$ | |
| 1.70 | 3 | " | " | $-S-(CH_2)_3-CH_3$ | 87-88 |
| 1.71 | 3 | " | Triaz | " | |
| 1.72 | 3 | " | Im | $-S-C(CH_3)_3$ | 128-129 |
| 1.73 | 3 | " | Im·$HNO_3$ | $-S-CH_2-$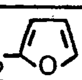 | 140-146 Zers. |
| 1.74 | 3 | " | Im | $-S-CH_2-$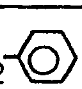 | 145-146 |
| 1.75 | 3 | " | Im | $-S-CH_2-$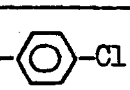 | 138-139 |
| 1.76 | 3 | " | Triaz | " | 165-166 |
| 1.77 | 3 | " | Im | $-S-CH_2-$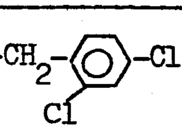 | 147-148 |

0237917

Tabelle 1 Fortsetzung

| Beisp.-Nr. | Variante | A | $Y_a$ | $Y_b$ | Schmp. [°C] |
|---|---|---|---|---|---|
| 1.78 | 3 | $C_6H_4$-4-Cl | Im | 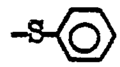 | |
| 1.79 | 3 | " | Im | 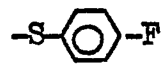 | |
| 1.80 | 3 | " | Im | 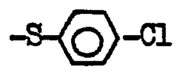 | 150-152 |
| 1.81 | 3 | " | Triaz | " | 131-133 |
| 1.82 | 3 | " | Im | 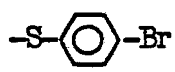 | |
| 1.83 | 3 | " | Im | 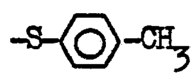 | |
| 1.84 | 3 | " | Im | 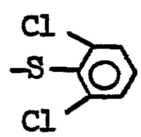 | |
| 1.85 | 3 | " | Im | 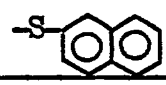 | 137 |
| 1.86 | 3 | " | Triaz | " | |
| 1.87 | 3 | $C_6H_3$-2,4-$Cl_2$ | Im | 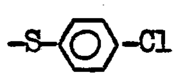 | 186-188 |
| 1.88 | 3 | $C_6H_4$-4-$CH_3$ | Im | " | 158-160 |
| 1.89 | 3 | $C_6H_4$-4-F | Im | " | 166-168 |
| 1.90 | 3 | $C_6H_4$-4-$OCH_3$ | Im | " | 161-162 |
| 1.91 | 3 | $C_6H_5$ | Im | " | 156-158 |
| 1.92 | 3 | $C_6H_4$-4-Cl | Im | 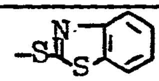 | Öl |
| 1.93 | 3 | " | Im | 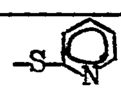 | 112-114 |

Tabelle 1 Fortsetzung

| Beisp.-Nr. | Variante | A | $Y_a$ | $Y_b$ | Schmp. [°C] |
|---|---|---|---|---|---|
| 1.94 | 3 | $C_6H_4$-4-Cl | Im | 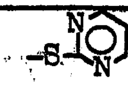 | 159-160 |
| 1.95 | 3 | " | Im | 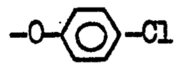 | 195-196 |
| 1.96 | 3 | " | Triaz | " | |
| 1.97 | 3 | " | Im | 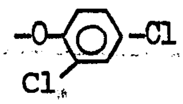 | |
| 1.98 | 3 | " | Im | 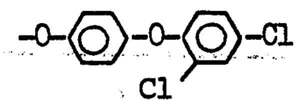 | 98-99 |
| 1.99 | 3 | " | Im | 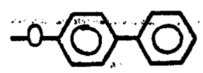 | 107-209 |
| 1.100 | 3 | " | Im | $O-C_2H_5$ | 139-140 |
| 1.101 | 3 | " | Im | $O-(CH_2)_3-CH_3$ | |
| 1.102 | 3 | " | Im | 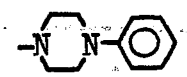 | Öl |
| 1.103 | 1 | $C_6H_3$-2,4-F,Cl | 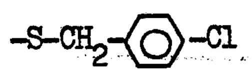 | Triaz | 130-131 |
| 1.104 | 1 | $C_6H_3$-2,4-Cl,F | " | Triaz | |
| 1.105 | 1 | $C_6H_4$-4-F | 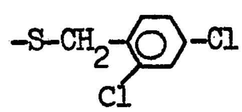 | Triaz | |
| 1.106 | 1 | " | 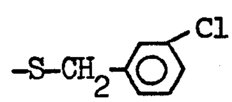 | Triaz | |
| 1.107 | 1 | " | 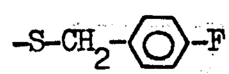 | Triaz | 180-183 |
| 1.108 | 1 | " | 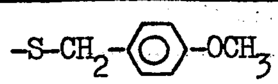 | Triaz | |

Tabelle 1 Fortsetzung

| Beisp.-Nr. | Variante | A | $Y_a$ | $Y_b$ | Schmp. [°C] |
|---|---|---|---|---|---|
| 1.109 | 1 | $-C_6H_4-4-F$ | 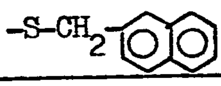 | Triaz | |
| 1.110 | 1 | " | 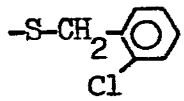 | Triaz | |
| 1.111 | 1 | " | 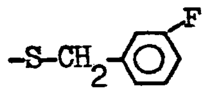 | Triaz | |
| 1.112 | 1 | " | 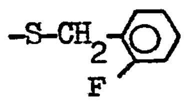 | Triaz | |
| 1.113 | 1 | " | 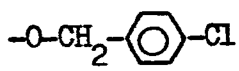 | Triaz | |
| 1.114 | 1 | " | 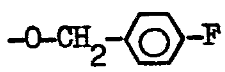 | Triaz | |
| 1.115 | 1 | $-C_6H_3-2,4-F_2$ | 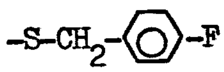 | Triaz | |
| 1.115 | 1 | " | 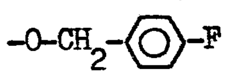 | Triaz | 102-4 |
| 1.116 | 1 | " | 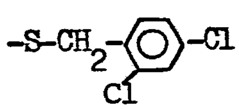 | Triaz | |
| 1.117 | 1 | $-C_6H_4-4-Cl$ | 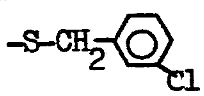 | Triaz | |
| 1.118 | 1 | " | 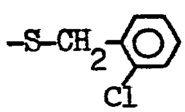 | Triaz | |
| 1.119 | 1 | " | 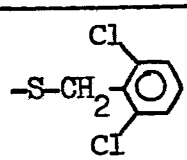 | Triaz | |
| 1.120 | 1 | 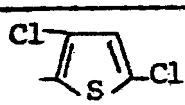 | Triaz | Triaz | 208-10 |
| 1.121 | 1 | " | 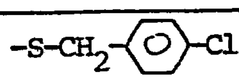 | Triaz | 75-77 |
| 1.122 | 1 | " | " | Im | |

Tabelle 1 Fortsetzung

| Beisp.-Nr. | Variante | A | $Y_a$ | $Y_b$ | Schmp. [°C] |
|---|---|---|---|---|---|
| 1.123 | 1 | $-C_6H_4-4-Cl$ | 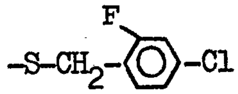 | Triaz | 160–162 |
| 1.124 | 1 | " | 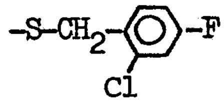 | Triaz | 163–165 |
| 1.125 | 1 | " | 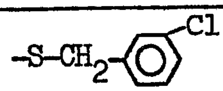 | Triaz | 136 |
| 1.126 | 1 | " | 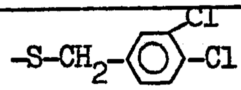 | Triaz | 127–129 |
| 1.127 | 1 | " | 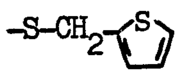 | " | 188–191 |
| 1.128 | 1 | " | 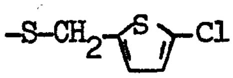 | " | 143–147 |
| 1.129 | 1 | " | 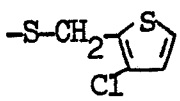 | " | 147–149 |
| 1.130 | 1 | " | 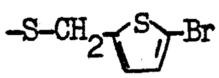 | " | 135–136 |
| 1.131 | 1 | " | 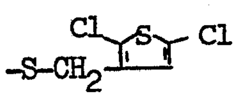 | " | 156–157 |
| 1.132 | 1 | " | 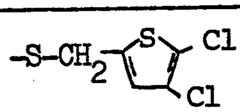 | " | 135–137 |
| 1.133 | 1 | " | 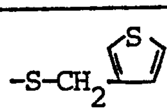 | " | 171–172 |

Tabelle 1 Fortsetzung

| Beisp.-Nr. | Variante | A | $Y_a$ | $Y_b$ | Schmp. [°C] |
|---|---|---|---|---|---|
| 1.134 | 1 | $-C_6H_4-4-Cl$ | 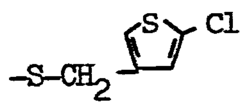 | Triaz | 140-141 |
| 1.135 | 1 | " | 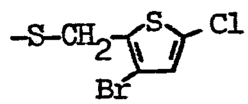 | " | 151-152 |
| 1.136 | 1 | " | 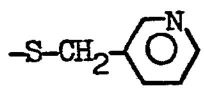 | " | 129-130 |
| 1.137 | 1 | " | 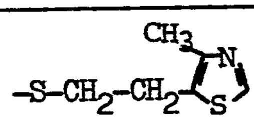 | " | 118-119 |
| 1.138 | 1 | " | 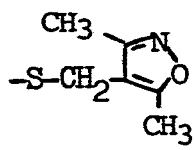 | " | 168-169 |
| 1.139 | 1 | " | 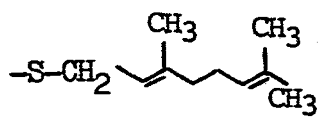 | " | 93-94 |
| 1.140 | 1 | " | 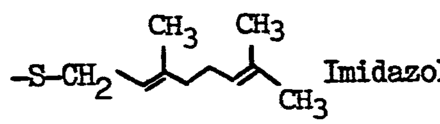 | Imidazol | Öl |
| 1.141 | 1 | " | 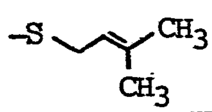 | Triazol | 144-145 |
| 1.142 | 1 | " | 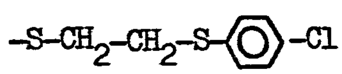 | " | 124-127 |
| 1.143 | 1 | " | 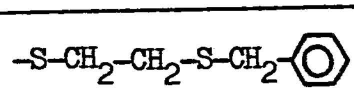 | " | 96-97 |

Tabelle 1 Fortsetzung

| Beisp.-Nr. | Variante | A | $Y_a$ | $Y_b$ | Schmp. [°C] |
|---|---|---|---|---|---|
| 1.144 | 1 | $-C_6H_4-4-F$ | 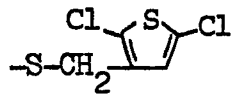 | Triazol | 112-113 |
| 1.145 | 1 | " | 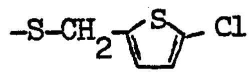 | " | 138-139 |
| 1.146 | 1 | " | 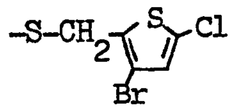 | " | 123-125 |
| 1.147 | 1 | " | 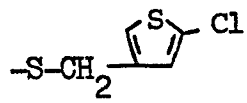 | " | 114-5 |
| 1.148 | 1 | " | 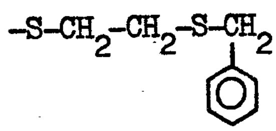 | " | 102-103 |
| 1.149 | 1 | " | 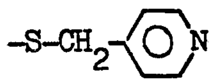 | " | 132-133 |
| 1.150 | 1 | " | 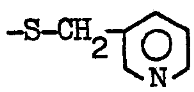 | Triaz | |
| 1.151 | 1 | $-C_6H_4-4-Cl$ | 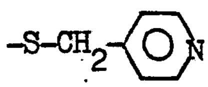 | " | 166-167 |
| 1.152 | 1 | " | 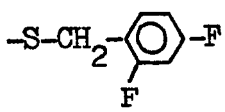 | Im | 150-152 |
| 1.153 | 1 | " | 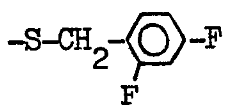 | Triaz | 137-139 |
| 1.154 | 1 | " | 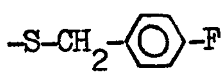 | Im | 170-171 |
| 1.155 | 1 | $-C_6H_4-4-F$ | 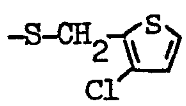 | Triaz | 151-152 |

Tabelle 1 Fortsetzung

| Beisp.-Nr. | Variante | A | $Y_a$ | $Y_b$ | Schmp. [°C] |
|---|---|---|---|---|---|
| 1.156 | 1 | $-C_6H_4-4-F$ | 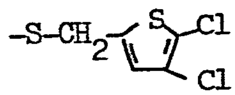 | Triaz | |
| 1.157 | 1 | $-C_6H_4-4-OCH_3$ | 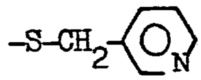 | Triaz | |
| 1.158 | 1 | $-C_6H_4-4-F$ | 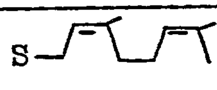 | " | 92-93 |
| 1.159 | 1 | $-C_6H_4-4-Cl$ | 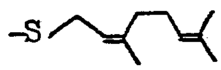 | " | 109-110 |
| 1.160 | 1 | " | 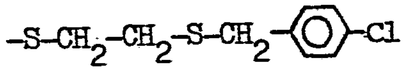 | " | 103-104 |
| 1.161 | 1 | $-C_6H_4-4-F$ | 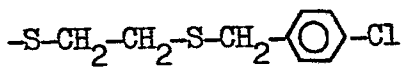 | " | 98-100 |
| 1.162 | 1 | $-C_6H_4-4-Cl$ | 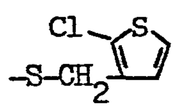 | " | |
| 1.163 | 1 | $-C_6H_4-4-F$ | 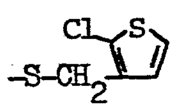 | " | |
| 1.164 | 1 | $-C_6H_4-4-Cl$ | 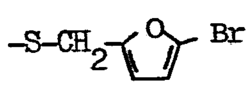 | " | 131-132 |
| 1.165 | 1 | " | 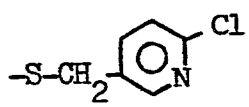 | " | |
| 1.166 | 1 | " | 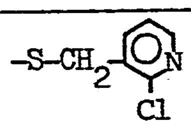 | " | |

Tabelle 1 Fortsetzung

| Beisp.-Nr. | Variante | A | $Y_a$ | $Y_b$ | Schmp. [°C] |
|---|---|---|---|---|---|
| 1.167 | 1 | $-C_6H_4-4-F$ | $-S-CH_2-$ 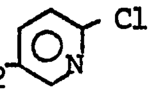 | Triaz | |
| 1.168 | 1 | $C_6H_4-4-OCH_3$ | $-S-CH_2$ 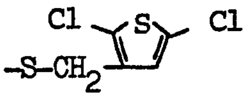 | " | |
| 1.169 | 1 | " | $-S-CH_2-$ 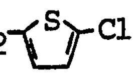 | " | |
| 1.170 | 1 | " | $-S-CH_2-$ 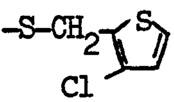 | " | 157-159 |
| 1.171 | 1 | $C_6H_4$-4-t-butyl | $-S-CH_2-$ 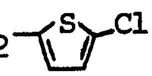 | " | 143-144 |
| 1.172 | 1 | " | $-S-CH_2-$ 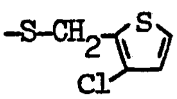 | " | |
| 1.173 | 1 | " | $-S-CH_2$ 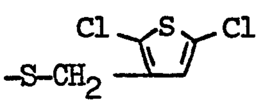 | " | |
| 1.174 | 1 | $-C_6H_4-4-Cl$ | $-S-CH_2-$ 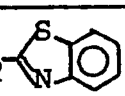 | " | |
| 1.175 | 1 | $-C_6H_4-4-Cl$ | $-S-CH_2-$ 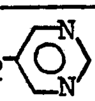 | " | |
| 1.176 | 1 | $-C_6H_4-4-F$ | $-S-CH_2-$ 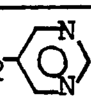 | " | |
| 1.177 | 1 | 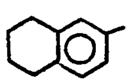 | $-S-CH_2-$ 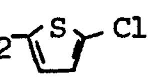 | " | 116-117 |

Tabelle 1 Fortsetzung

| Beisp.-Nr. | Variante | A | $Y_a$ | $Y_b$ | Schmp. [°C] |
|---|---|---|---|---|---|
| 1.178 | 1 | 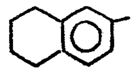 | 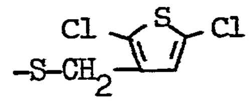 | Triaz | |
| 1.179 | 1 | 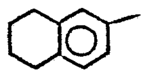 | 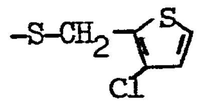 | " | |
| 1.180 | 1 | 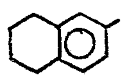 | 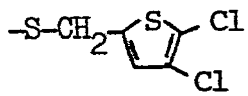 | " | |
| 1.181 | 1 | 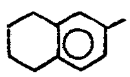 | 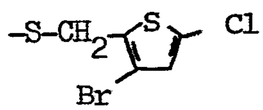 | " | |
| 1.182 | 1 | 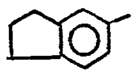 | 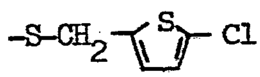 | " | 146-147 |
| 1.183 | 1 | 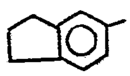 | 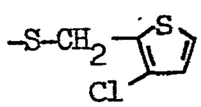 | " | 130-131 |
| 1.184 | 1 | 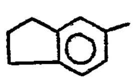 | 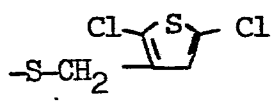 | " | 148-150 |
| 1.185 | 1 | 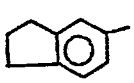 | 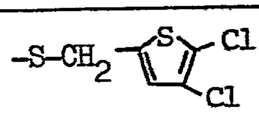 | " | |
| 1.186 | 1 | $-C_6H_4-4-Cl$ | 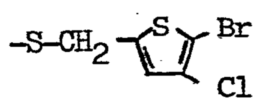 | " | |
| 1.187 | 1 | $-C_6H_3-2,4-F_2$ | 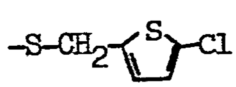 | " | |
| 1.188 | 1 | $-C_6H_3-2,4-F_2$ | 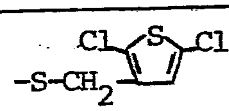 | " | |
| 1.189 | 1 | " | 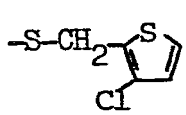 | " | |

Tabelle 1 Fortsetzung

| Beisp.-Nr. | Variante | A | $Y_a$ | $Y_b$ | Schmp. [°C] |
|---|---|---|---|---|---|
| 1.190 | 1 | $-C_6H_4-4-Cl$ | 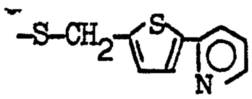 | Triaz | |
| 1.191 | 1 | $-C_6H_4-4-CH_3$ | 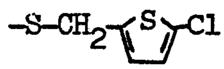 | " | |
| 1.192 | 1 | $-C_6H_4-4-CH_3$ | 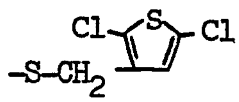 | " | |
| 1.193 | 1 | 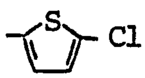 | 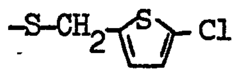 | " | |
| 1.194 | 1 | 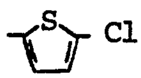 | 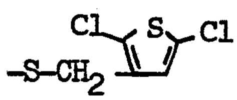 | " | |
| 1.195 | 1 | 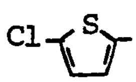 | 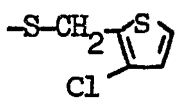 | " | |
| 1.196 | 1 | $-C_6H_4-4-Cl$ | 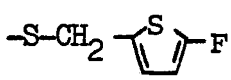 | " | |
| 1.197 | 1 | 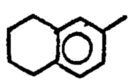 | 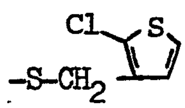 | " | |
| 1.198 | 1 | $-C_6H_4-4-Cl$ | 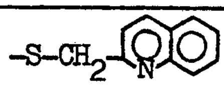 | " | 143-145 |
| 1.199 | 1 | 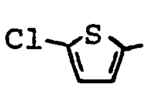 | 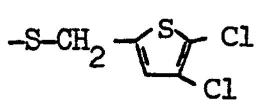 | " | |
| 1.200 | 1 | $-C_6H_4-4-Cl$ | 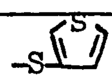 | " | |

Tabelle 1 Fortsetzung

| Beisp.-Nr. | Variante | A | $Y_a$ | $Y_b$ | Schmp. [°C] |
|---|---|---|---|---|---|
| 1.201 | 1 | $-C_6H_4-4-Cl$ | 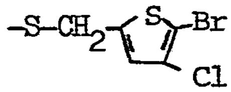 | Triaz | |
| 1.202 | 1 | $-C_6H_4-4-F$ | 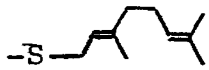 | " | Öl |
| 1.203 | 1 | 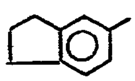 | 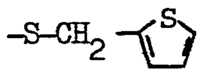 | " | 146-147 |
| 1.204 | 1 | 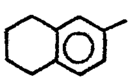 | 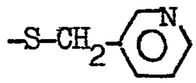 | " | 130-131 |
| 1.205 | 1 | $-C_6H_3-2,4-F_2$ | 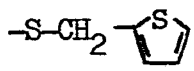 | " | |
| 1.206 | 1 | 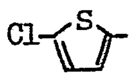 | 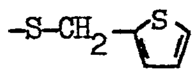 | " | |
| 1.207 | 1 | $-C_6H_4-4-CH_3$ | 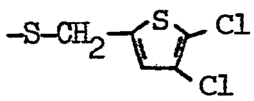 | " | |
| 1.208 | 1 | 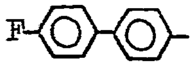 | 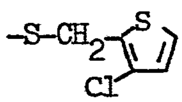 | " | |
| 1.209 | 1 | 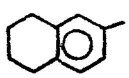 | 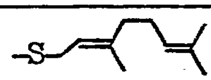 | " | |
| 1.210 | 1 | 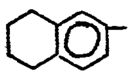 | 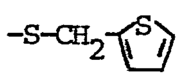 | " | 144-145 |
| 1.211 | 1 | $-C_6H_4-4-CH_3$ | 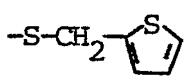 | " | 156-157 |
| 1.212 | 1 | $-C_6H_4-4-CH_3$ | 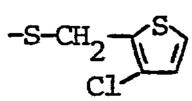 | " | 153-155 |
| 1.213 | 1 | $-C_6H_4-4-OCH_3$ | 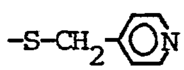 | | 128-129 |

Beispiel 5

1-[1-(4-Chlorphenyl)-1-methoxy-2-(imidazol-1-yl)-ethyl]-1-(4-chlorphenoxy)-cyclopropan

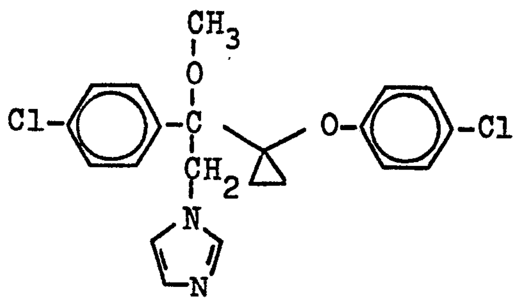

Zur Suspension aus 0,66 g = 22 mmol Natriumhydrid (80%ige Suspension in Öl) in 20 ml trockenem Dimethylformamid wurden portionsweise bei 20°C 7,8 g = 20 mmol 1-[1-(4-Chlorphenyl)-1-hydroxy-2-(imidazol-1-yl)-ethyl]-1-(4-chlorphenoxy)-cyclopropan (Verbindung aus Beispiel 2) eingetragen und bis zum Ende der Wasserstoffentwicklung nachgerührt. Anschließend tropfte man die Lösung von 3,1 g = 22 mmol Methyljodid in 5 ml trockenem Dimethylformamid bei 0°C zu, ließ auf Zimmertemperatur aufwärmen, rührte 15 Stunden nach und arbeitete auf, wie in Beispiel 1 angegeben. Der harzige Rückstand (9,3 g) wurde über eine Kieselgel-Säule mit Methylenchlorid:Ethanol = 20:1 als Elutionsmittel gereinigt. Der Rückstand der einheitlichen Fraktionen kristallisierte nach Auflösen in Ether. Man erhielt 4,4 g (55 % d.Th.) vom Schmp.: 117 - 119°C.

Beispiel 6

Analog Beispiel 5, jedoch unter Verwendung entsprechender Verbindungen der Formel I mit Z= OH und einem Alkyl- oder Benzylhalogenid können auch die in Tabelle 2 aufgeführten Verbindungen der Formel I hergestellt werden.

Im, Triaz, a und b haben die gleiche Bedeutung wie zur Tabelle 1 angegeben.

Tabelle 2

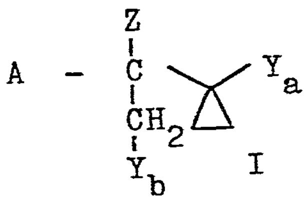

I

| Beisp. Nr. | hergest. aus | A | $Y_a$ | $Y_b$ | Z | Schmp.: [°C] |
|---|---|---|---|---|---|---|
| 2.1 | 1.29 | $-C_6H_4$-4-Cl | 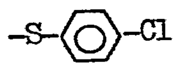 | Triaz | $-OCH_3$ | 148–150 |
| 2.2 | 1.30 | " | 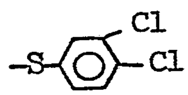 | Im | " | Öl |
| 2.3 | 1.31 | " | 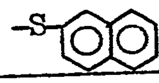 | Im | " | Öl |
| 2.4 | 1.35 | " | 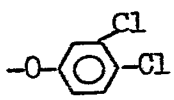 | Im | " | Öl |
| 2.5 | 1.38 | " | 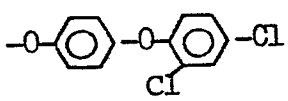 | Im | " | 180–182 |
| 2.6 | 1.11 | " | 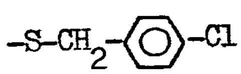 | Triaz | " | Öl |
| 2.7 | 1.80 | " | Im | 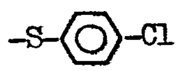 | " | 118–120 |
| 2.8 | 1.56 | " | Im | Im | 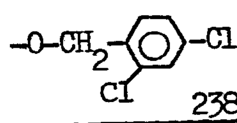 | 238–240 |
| 2.9 | 1.56 | " | " | " | 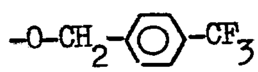 | 182–187 |
| 2.10 | 1.62 | 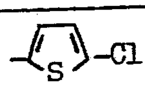 | Triaz | Triaz | $OCH_3$ | |
| 2.11 | 1.20 | $-C_6H_4$-4-F | 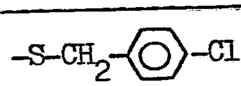 | Triaz | $OCH_3$ | |
| 2.12 | 1.12 | $-C_6H_4$-4-Cl | 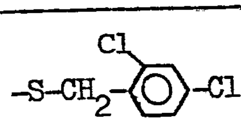 | Triaz | $OCH_3$ | |

Beispiel 7

1-[1-(4-Chlorphenyl)-1-chlor-2-(1,2,4-triazol-1-yl)-ethyl]-1-(4-chlorbenzylthio)-cyclopropan

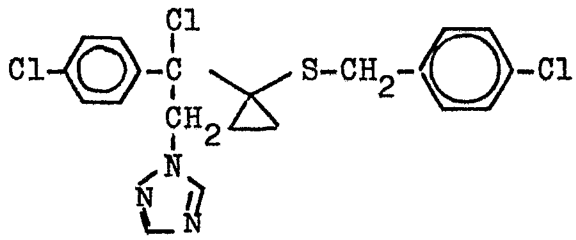

Zur Lösung von 4 g = 30 mmol Thionylchlorid in 30 ml trockenem Acetonitril tropfte man unter Rühren und Kühlen bei 0°C die Lösung von 4,2 g = 10 mmol 1-[1-(4-Chlorphenyl)-1-hydroxy-2-(1,2,4-triazol-1-yl)-ethyl]-1-(4-chlorbenzylthio)-cyclopropan (Verbindung 1.11, Tab. 1) in 20 ml trockenem Methylenchlorid, rührte 15 Stunden bei 20°C und eine Stunde am Rückfluß. Anschließend wurde im Wasserstrahlvakuum eingeengt, der Rückstand in 100 ml Methylenchlorid/30 ml 2n NaOH gelöst, aufgearbeitet wie in Beispiel 1 angegeben und säulenchromatographiert wie in Beispiel 5 angegeben. Man erhielt 1 g (22 % d.Th.) vom Schmp.: 88 - 92°C

Beispiel 8

1-(4-Chlorphenyl)-1-[1-(imidazol-1-yl)-cyclopropan-1-yl]-1-hydroxyeth-2-yl-benzylsulfoxid

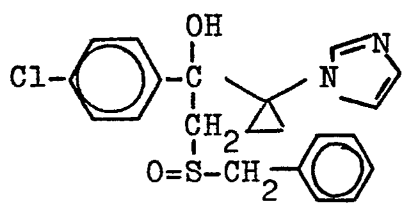

Zur Lösung von 3,8 g = 10 mmol 1-[1-(4-Chlorphenyl)-1-hydroxy-2-benzylthio-ethyl]-1-(imidazol-1-yl)-cyclopropan (Verbindung 1.74, Tab. 1) in 20 ml Methylenchlorid tropfte man unter Rühren und Kühlen bei 0°C die Lösung von 1 g = 10 mmol 35 %igem Wasserstoffperoxid in 5 ml Eisessig,

ließ innerhalb 3h auf 20°C aufwärmen, rührte 15 Stunden nach und arbeitete auf, wie im Beispiel 1 angegeben. Der harzige Rückstand kristallisierte nach mehrtätigem Stehen aus Essigsäureethylester. Man erhielt 2,8 g (70% d.Th.) vom Schmp.: 205 - 207°C. (Methanol).

Beispiel 9

Analog Beispiel 8 können auch die in Tabelle 3 aufgeführten Verbindungen der Formel I hergestellt werden. Im, Triaz, a und b haben die gleiche Bedeutung wie zur Tabelle 1 angegeben.

Tabelle 3

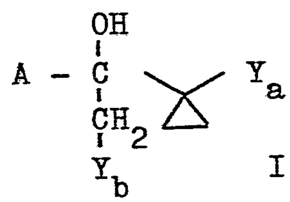

I (mit Z= OH)

| Beisp. Nr. | hergest. aus | A | $Y_a$ | $Y_b$ | Schmp. [°C] |
|---|---|---|---|---|---|
| 3.1 | 1.11 | $-C_6H_4-4-Cl$ | 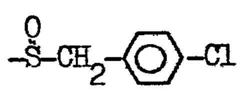 | Triaz | 1) 208-209 a)<br>2) 197-198 |
| 3.2 | 1.21 | $-C_6H_4-4-F$ | " | Triaz | |
| 3.3 | 1.16 | $-C_6H_3-2,4-F_2$ | " | Triaz | |
| 3.4 | 1.44 | 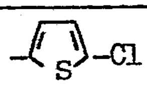 | " | Triaz | |
| 3.5 | 1.15 | $-C_6H_3-2,4-Cl_2$ | " | Triaz | |
| 3.6 | 1.103 | $-C_6H_3-2.4-F,Cl$ | " | Triaz | |
| 3.7 | 1.104 | $-C_6H_3-2.4-Cl,F$ | " | Triaz | |

a) Die Diastereomerenpaare der Sulfoxide wurden säulenchromatographisch getrennt, wie in Beispiel 5 angegeben. Das Verhältnis beträgt 1) zu 2) wie 1:3, wobei 1) die unpolarere Komponente darstellt und zuerst von der Säule eluiert wird.

Tabelle 3 (Fortsetzung)

| Beisp. Nr. | hergest. aus | A | $Y_a$ | $Y_b$ | Schmp. [°C] |
|---|---|---|---|---|---|
| 3.8 | 1.13 | $-C_6H_4-4-Cl$ | 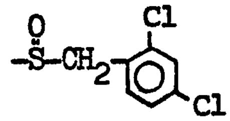 | Triaz | 1) 210-214<br>2) 201-204 |
| 3.9 | 1.18 | $-C_6H_4-4-Cl$ | 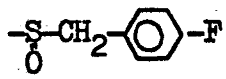 | Triaz | 1) 175<br>2) 189-190 |
| 3.10 | 1.127 | $-C_6H_4-4-Cl$ | 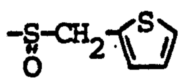 | Triaz | 1) Öl<br>2) 179-181 |
| 3.11 | 1.129 | " | 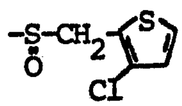 | " | |

Beispiel 10

1-[1-(4-Chlorphenyl)-1-hydroxy-2-(1,2,4-triazol-1-yl)-ethyl]-1-(4-chlorbenzylsulfonyl)-cyclopropan

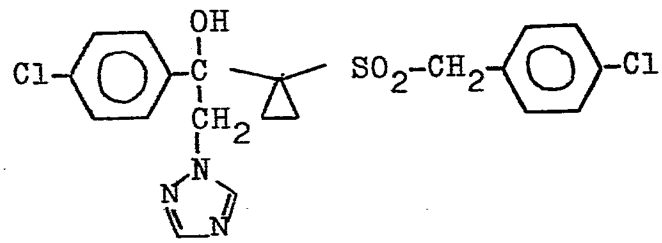

Zur Lösung von 4,2 g = 10 mmol 1-[1-(4-Chlorphenyl)-1-hydroxy-2-(1,2,4-triazol-1-yl)-ethyl]-1-(4-chlorbenzylthio)-cyclopropan (Verbindung 1.11, Tab. 1) in 30 ml Eisessig tropfte man unter Rühren bei 20°C 2,2 g = 22 mmol 35 %iges Wasserstoffperoxid zu, rührte anschließend 2h bei 20°C, 4h am Rückfluß und arbeitete auf, wie in Beispiel 1 angegeben. Man erhielt 2,9 g (65 % d.Th.) vom Schmp.: 189 - 190°C.

Beispiel 11

Analog Beispiel 10 können auch die in Tabelle 4 angeführten Verbindungen der Formel I hergestellt werden.

Im, Triaz, a und b haben die gleiche Bedeutung wie zur Tabelle 1 angegeben.

Tabelle 4

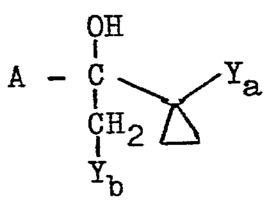

I (mit Z = OH)

| Beisp. Nr. | hergest. aus | A | $Y_a$ | $Y_b$ | Schmp. [°C] |
|---|---|---|---|---|---|
| 4.1 | 1.21 | $-C_6H_4$-4-F | 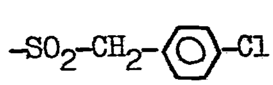 | Triaz | |

Forts. Tabelle 4

| Beisp. Nr. | hergest. aus | A | $Y_a$ | $Y_b$ | Schmp. [°C] |
|---|---|---|---|---|---|
| 4.2 | 1.16 | $-C_6H_3-2,4-F_2$ | " | Triaz | |
| 4.3 | 1.44 | 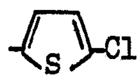 | " | Triaz | |
| 4.4 | 1.15 | $-C_6H_3-2,4-Cl_2$ | " | Triaz | |
| 4.5 | 1.103 | $-C_6H_3-2,4-F,Cl$ | " | Triaz | |

Beispiele für das zweite Herstellverfahren nach Verfahrensvariante b

Beispiel 12

Herstellung einer Verbindung der Formel V

1-(4-Chlorphenyl)-1-[1-(4-chlorphenoxy)-cyclopropan-1-yl]-ethen.

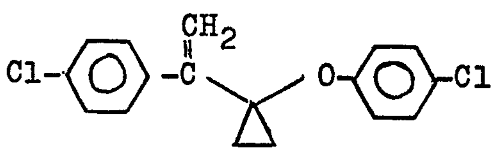

Die Suspension aus 3 g = 0,1 mol Natriumhydrid (80 %ige Suspension in Öl) und 50 ml trockenem Dimethylsulfoxid wurde unter Stickstoffatmosphäre bei 60 - 70°C gerührt bis die Wasserstoffentwicklung beendet war (ca. 1.5 Stunden). Anschließend tropfte man bei -10°C die Lösung von 40,4 g = 0,1 mol Methyltriphenylphosphoniumjodid in 100 ml trockenem Dimethylsulfoxid zu, ließ auf 20°C aufwärmen, tropfte die Lösung von 30,7 g = 0,1 mol 1-(4-Chlorbenzoyl)-1-(4-chlorphenoxy)-cyclopropan in 30 ml trockenem Dimethylsulfoxid zu, rührte eine Stunde bei 20°C, 4 Stunden bei 60°C und arbeitete auf, wie in Beispiel 1 angegeben. Den Rückstand löste man in der Siedehitze im gleichen Volumen Essigsäureethylester, kühlte mit einem Eisbad ab, saugte den Niederschlag ab und wusch mit Ether nach. Die organische Phase wurde im Wasserstrahlvakuum eingeengt, der Rückstand dreimal mit 100 ml Petrolether geschüttelt und abdekantiert. Die Petroletherphase wurde über neutrales Aluminiumoxid filtriert und im Vakuum eingeengt. Man erhielt 26 g Öl, die ohne weitere Reinigung in Beispiel 14 verwendet wurden.

Beispiel 13
1-(4-Chlorphenyl)-1-[1-(imidazol-1-yl)-cyclopropan-1-yl]-ethen

Gemäß Beispiel 12, jedoch unter Verwendung von 1-(4-Chlorbenzoyl)-1-(imidazol-1-yl)-cyclopropan erhielt man 14,7 g) (60 % d.Th.) vom Schmp.: 94 - 95°C.

Beispiel 14
Herstellung einer Verbindung der Formel IV

2-(4-Chlorphenyl)-2-[1-(4-chlorphenoxy)-cyclopropan-1-yl]-oxiran (vgl. Beispiel 2a).

Zur Lösung von 6,1 g = 20 mmol 1-(4-Chlorphenyl)-1-[1-(4-chlorphenoxy)-cyclopropan-1-yl]-ethen (Verbindung aus Beispiel 12) in 20 ml Chloroform tropfte man unter Rühren bei 20°C die Lösung 4,3 g = 25 mmol 80 %iger m-Chlorperbenzoesäure in 100 ml Chloroform, rührte 15 Stunden bei 20°C und 3 Stunden am Rückfluß. Zur Aufarbeitung schüttelte man die Mischung dreimal mit 50 ml 2n Natronlauge, einmal mit 2n Natriumbisulfitlösung, wusch mit Wasser, trocknete über Natriumsulfat und engte die Lösung im Wasserstrahlvakuum ein. Den Rückstand löste man im doppelten Volumen Ether.

Nach Animpfen mit einem Kristall aus Beispiel 2a kristallisierten 3,2 g vom Schmp. 96 - 97°C.

Beispiel 15

2-(4-Chlorphenyl)-2-[1-(imidazol-1-yl)-cyclopropan-1-yl]-oxiran

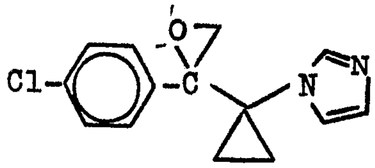

Gemäß Beispiel 14, jedoch unter Verwendung von 4,9 g = 20 mmol 1-(4-Chlorbenzoyl)-1-(imidazol-1-yl)-cyclopropan erhielt man 4,5 g Öl, das ohne weitere Reinigung in Beispiel 17 verwendet wurde.

Beispiel 16

Herstellung einer Verbindung der Formel I
1-[1-(4-Chlorphenyl)-1-hydroxy-2-(imidazol-1-yl)-ethyl]-1-(4-chlorphenoxy)-cyclopropan

3,2 g = 10 mmol 2-(4-Chlorphenyl)-2-[1-(4-chlorphenoxy)-cyclopropan-1-yl]-oxiran (Verbindung aus Beispiel 14) setzte man mit 2,04 g = 30 mmol Imidazol in Substanz um, wie in Beispiel 2a) angegeben, und erhielt 2,9 g (75 % d.Th.) vom Schmp.: 172 - 173°C

Beispiel 17

1-[1-(4-Chlorphenyl)-1-hydroxy-2-(4-chlorphenylthio)-ethyl]-1-(imidazol-1-yl)-cyclopropan

Zur Lösung von 4,5 g = 17,2 mmol 2-(4-Chlorphenyl)-2-[1-(imidazol-1-yl)-cyclopropan-1-yl]-oxiran (Verbindung aus Beispiel 15) und 1,7 g = 17 mmol Triethylamin in 20 ml Aceton tropfte man unter Rühren und Stickstoffatmosphäre bei 20°C die Lösung von 2,4 g = 17 mmol p-Chlorthiophenol in 10 ml Aceton, rührte 2 Stunden bei 20°C, 5 Stunden am Rückfluß und arbeitete auf, wie in Beispiel 1 angegeben.

Man erhielt 3,1 g (45 % d.Th.) vom Schmp.: 148 - 150°C (aus Methanol).

Als Beispiel für die hohe orale und parenterale in-vivo-Wirksamkeit der erfindungsgemäßen Verbindungen werden Behandlungsergebnisse an experimentell mit Candida albicans infizierten Labortieren angeführt.

Zur Feststellung der oralen und parenteralen Wirksamkeit
4 wurden Gruppen von je 5 18-20 g schweren Mäusen (Stamm HOE: NMRKF; SPF 71) mit $2 \cdot 10^{6}$ Keimen/Tier infiziert.

Die Behandlung der Tiere erfolgte oral bzw. subcutan in 8 gleichen Einzeldosen zu je 30 mg/kg, 10 mg/kg bzw. 2,5 mg/kg Körpergewicht (-24/-18/-2h/+2/24/30/48/54h).

Neben der mit den erfindungsgemäßen Substanzen I behandelten Gruppe von 5 Tieren wurde zum Vergleich eine Gruppe von ebenfalls 5 Tieren mit den Referenzsubstanzen Ketoconazol bzw. Fluconazol behandelt. Eine Kontrollgruppe von 10 Tieren blieb nach der Infektion unbehandelt.

Wie aus Tabelle 2 hervorgeht, fand sich bei den erfindungsgemäßen Verbindungen 1.11 und 1.21 eine bis um das Doppelte verlängerte Überlebenszeit der Tiere nach der Infektion, verglichen mit dem derzeitigen Standardpräparat Ketoconazol.

Bei Verbindungen 1.131 und 1.151 wird Fluconazol als Standard verwendet, wobei sich eine bis zu 50 % erhöhte Überlebenszeit der Tiere ergab.

Tabelle 5

| Dosis | Präparat Beispiel Nr. | Anzahl Tiere | Überlebenszeiten Tage nach Infektion | x Tage | Überlebenszeit in % (Standardpräp.= 100 %) |
|---|---|---|---|---|---|
| oral | 1.11 | 10 | 8 8 8 9 9 9 10 10 12 25 | 10,8 | 308,5 |
| 8 x 10 mg/kg | 1.21 | 10 | 8 8 9 9 9 9 9 10 11 12 | 8,9 | 254,2 |
| | Ketoconazol | 10 | 2 3 3 3 3 4 4 4 4 5 | 3,5 | 100 |
| oral | 1.131 | 10 | 7 8 8 8 9 9 11 13 15 16 | 10,4 | 118,2 |
| 8 x 2,5 mg/kg | 1.151 | 10 | 9 9 10 10 13 15 15 17 18 18 | 13,4 | 152,3 |
| | Fluconazol | 10 | 7 7 7 8 8 8 10 11 11 11 | 8,8 | 100 |
| subcutan | 1.11 | 10 | 19 21 21 23 26 27 27 29 29 29 | 25,1 | 398,4 |
| 8 x 10 mg/kg | 1.21 | 10 | 13 13 20 20 20 21 26 28 31 33 | 22,5 | 357,1 |
| | Ketoconazol | 10 | 5 5 5 6 6 6 6 7 8 9 | 6,3 | 100 |
| Kontrollen infiz. Tiere unbehandelt | - | 10 | 1 1 2 2 2 2 2 2 3 3 | 2,0 | - |

PATENTANSPRÜCHE:

1. Verbindungen der Formel I

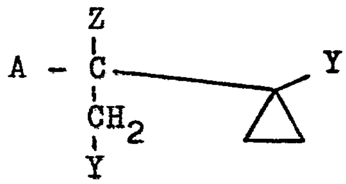 I,

worin bedeuten:

A t-Butyl, Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Furyl, Pyridyl, Isoxazolyl, Pyrazolyl, Benzofuryl, Benzothienyl,

wobei die genannten Ringsysteme unsubstituiert oder mit 1-3 Substituenten, die gleich oder verschieden sind, substituiert sind, welche F, Cl, Br, J, $(C_1-C_8)$-Alkyl, geradkettig oder verzweigt und unsubstituiert oder mit 1-9 F- oder Cl-Atomen substituiert, $(C_3-C_8)$-Cycloalkyl, $(C_1-C_8)$-Alkoxy, geradkettig oder verzweigt, und unsubstituiert oder mit 1-9 F- oder Cl-Atomen substituiert, $(C_3-C_8)$Cycloalkyl-$(C_1-C_4)$Alkyl, $(C_1-C_8)$Alkylthio, $(C_1-C_8)$-Alkylsulfinyl- und -sulfonyl, $NO_2$, CN sind;

Y

1) 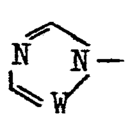

mit W= CH oder N

2) $- X - R^1$

mit X = -O-, -S-, >S=O, >$SO_2$ und

$R^1$ = $(C_1-C_{12})$-Alkyl (geradkettig oder verzweigt, unsubstituiert oder durch 1 bis 3 F-, Cl- oder Br-Atome oder $CH_3$-Gruppen substituiert), $(C_2-C_{20})$-Alkenyl (geradkettig oder verzweigt; ein- oder mehrfach ungesättigt, in Form der reinen e- oder z-Isomeren oder der e/z-Diastereomerengemische, unsubstituiert oder durch 1 bis 3 F-, Cl- oder Br-Atome oder $CH_3O$-Gruppen substituiert), $(C_2-C_{20})$-Alkinyl (geradkettig oder verzweigt; unsubstituiert oder durch 1 bis 3 F-, Cl- oder Br-Atome oder $CH_3O$-Gruppen substituiert), $(C_2-C_{20})$Alkeninyl (geradkettig oder verzweigt; ein- oder mehrfach ungesättigt, in Form der reinen e- oder z-Isomeren oder der e/z-Diastereomerengemische, unsubstituiert oder durch 1 bis 3 F-, Cl- oder Br-Atome oder $CH_3O$-Gruppen substituiert), $(C_3-C_8)$-Cycloalkyl, $(C_3-C_8)$Cycloalkyl-$(C_1-C_4)$-alkyl, Phenyl, Biphenylyl, Phenoxyphenyl, Phenylthiophenyl, Phenyl$(C_1-C_4)$-alkyl, Naphthyl, Biphenylyl$(C_1-C_4)$-alkyl, Phenylthiophenyl$(C_1-C_4)$-alkyl, Phenoxyphenyl-$(C_1-C_4)$alkyl, Naphthyl$(C_1-C_4)$-alkyl, Benzothiazol-2-yl, Benzimidazol-2-yl, N-$(C_1-C_4)$-Alkylbenzimidazol-2-yl, Benzyl, Pyridyl, Pyrid-2-yl-methyl, Pyrid-3-yl-methyl, Pyrid-4-yl-methyl, Pyrimidin-2-yl, Pyrimidin-2-yl-methyl, Pyrimidin-4-yl-methyl, Pyrimidin-5-yl-methyl, Furfuryl, Thien-2-yl, Thien-3-yl, Thien-2-yl-methyl, Thien-3-yl-methyl, Isoxazol-4-yl-methyl, Thiazol-2-yl-methyl, Thiazol-5-yl-methyl, Thiazol-5-yl-eth-2-yl, $(C_2-C_3)$-Alkyl- X-aryl (mit X wie vorstehend definiert und Aryl

gleich Phenyl, Benzyl, Thien-2-yl-methyl, Thien-3-yl-methyl), Benzothiazol-2-yl-methyl, Chinolinyl-methyl, Pyridyl-phenyl-methyl, wobei die genannten Ringsysteme unsubstituiert oder durch 1, 2 oder 3 Substituenten, die gleich oder verschieden sind und jeweils F, Cl, Br, J, $CF_3$, $(C_1-C_4)$-Alkyl, $(C_1-C_4)$-Alkoxy bedeuten, substituiert sind; die Ringsysteme Pyridiminyl und Thienyl jedoch auch durch einen Phenylrest, der unsubstituiert oder seinerseits wie vorstehend angegeben substituiert ist,

3) 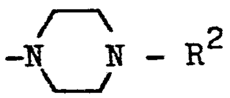

mit $R^2$= $(C_1-C_8)$-Alkyl, $(C_5-C_8)$-Cycloalkyl, Acetyl, Phenyl, Benzyl; wobei der Phenylkern jeweils unsubstituiert oder mit 1-3 F, Cl, Br, J, $CF_3$, $(C_1-C_4)$-Alkyl- oder $(C_1-C_4)$-Alkoxygruppen substituiert ist,

4) 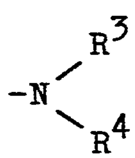 mit $R^3$ und $R^4$ gleich oder verschieden $(C_1-C_4)$Alkyl,

5) 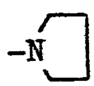 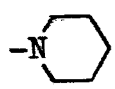 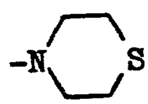 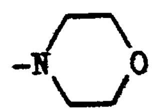

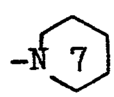 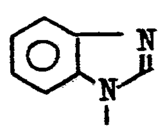 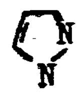

wobei mindestens eine der beiden Gruppen Y die Bedeutung von Y 1) hat;

Z = OH, $(C_1-C_4)$-Alkylcarbonyloxy, F, Cl, Br, $(C_1-C_4)$-Alkoxy, Benzyloxy, unsubstituiert oder 1-2fach substituiert mit F, Cl, Br, $CF_3$;

und ihre Salze mit physiologisch verträglichen Säuren

mit Ausnahme derjenigen Verbindungen, in denen gleichzeitig

a) beide Y die Bedeutung von Y 1) mit W=N und

b) A die Bedeutung Phenyl, gegebenenfalls substituiert mit 1-3 Substituenten, die unabhängig voneinander ausgewählt sind aus F, Cl, Br, J, $CF_3$, $(C_1-C_4)$-Alkyl und $(C_1-C_4)$-Alkoxy, oder 5-Chlorpyrid-2-yl und

c) Z die Bedeutung wie oben angegeben

haben.

2. Verbindung der Formel I nach Anspruch 1, dadurch gekennzeichnet, daß mindestens einer der Substituenten folgende Bedeutung hat:

A: Phenyl, Biphenylyl, 1,2,3,4-Tetrahydronaphthyl, Thienyl, Indanyl, jeweils unsubstituiert oder im Ring mit ein oder zwei Substituenten, die gleich oder verschieden sind und jeweils F, Cl, Br, $CF_3$, $(C_1-C_4)$-Alkyl oder $(C_1-C_4)$-Alkoxy bedeuten, substituiert sind,

Y: 1) 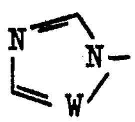 mit W= CH oder N

2) $-X-R^1$ mit

X = O, S

$R^1$ = $(C_1-C_{15})$-Alkyl, geradkettig oder verzweigt, $(C_2-C_{15})$-Alkenyl, geradkettig oder verzweigt, ein- oder mehrfach ungesättigt, Phenyl, Biphenylyl, Phenoxyphenyl, Phenylthiophenyl, Phenyl-$(C_1-C_2)$alkyl, Naphthyl, Biphenylyl$(C_1-C_2)$alkyl, Naphthyl$(C_1-C_2)$alkyl, Benzothiazol-2-yl, Benzimidazol-2-yl, Furfuryl, Thien-2-yl-methyl, Thieny-3-yl-methyl, Isoxazol-4-yl-methyl, Pyrid-3-yl-methyl, Pyrid-4-yl-methyl, Ethyl-thio-aryl mit Aryl gleich Phenyl,

Benzyl, Thien-2-ylmethyl, Thien-3-yl-methyl, wobei die genannten Ringsysteme unsubstituiert oder durch 1, 2 oder 3 Substituenten, die gleich oder verschieden sind und jeweils F, Cl, Br, $CF_3$, Methyl, Methoxy-Gruppen bedeuten, substituiert sind,

3.) 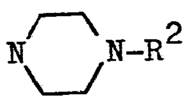 mit

$R^2$= Phenyl, Benzyl, jeweils unsubstituiert oder mit 1 oder 2 F, Cl, Br, $CF_3$, Methyl oder Methoxy-Gruppen substituiert,

Z = OH, $OCH_3$, F, Cl
wobei mindestens eine der beiden Gruppen Y die Bedeutung von Y 1) hat,
und ihre Salze mit physiologisch verträglichen Säuren.

3. Verbindung I nach Anspruch 1, dadurch gekennzeichnet, daß mindestens einer der Substituenten folgende Bedeutung hat:

A Phenyl, Thienyl, jeweils unsubstituiert oder substituiert durch 1 oder 2 F- oder Cl-Atome, Methyl, Methoxy

Y 1) 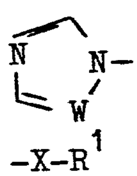 mit W = CH oder N

2) $-X-R^1$ mit X = O, S
$R^1$ = $(C_1-C_{12})$-Alkyl (geradkettig oder verzweigt), Geranyl, Neryl, Phenyl, Benzyl, Naphthyl, Thien-2-yl-methyl Thien-3-yl-methyl, Isoxazol-4-yl-methyl, Pyrid-3-yl-methyl, Pyrid-4-yl-methyl, jeweils unsubstituiert oder 1- oder 2-fach substituiert durch F, Cl, Methyl, Methoxy

Z = OH
wobei mindestens eine der beiden Gruppen Y die Bedeutung von Y 1) hat,
und ihre Salze mit physiologisch verträglichen Säuren.

4. Verfahren zur Herstellung einer Verbindung I nach Anspruch 1, dadurch gekennzeichnet, daß man

a) eine Verbindung der Formel II

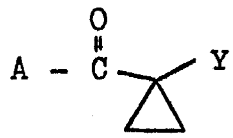

II,

in welcher A und Y die in Anspruch 1 genannten Bedeutungen haben,
mit einem Schwefelylid der Formel III

$$(CH_3)_2\underset{(O)_p}{\overset{}{S}}CH_2 \qquad III,$$

worin p Null oder 1 bedeutet,
zu einer Verbindung der Formel IV

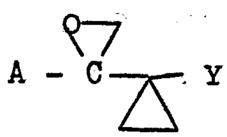

IV

umsetzt,
und anschließend die Verbindung IV mit einem Nukleophil der Formel Y-M umsetzt, in welcher
Y die in Anspruch 1 genannte Bedeutung hat und
M Wasserstoff oder ein Metalläquivalent ist,
wobei eine Verbindung I mit Z= OH entsteht, und, falls gewünscht, diese Verbindung I acyliert, alkyliert oder in eine Verbindung mit Z= F, Cl oder Br überführt, und gegebenenfalls am Schwefel einer Thioethergruppe zum Sulfoxid oder Sulfon oxidiert;

b) eine Verbindung der Formel II

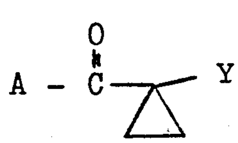

II,

in welcher A und Y die in Anspruch 1 genannten Bedeutungen haben, mit einem Phosphorylid zu einer Verbindung der Formel V

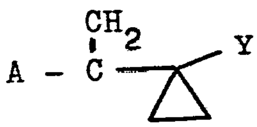

V

umsetzt,

und anschließend die Verbindung V mit einem organischen Peroxid zu einer Verbindung der Formel IV

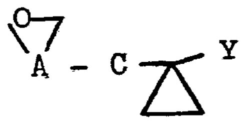

IV

umsetzt,

und anschließend die Verbindung IV mit einem Nucleophil der Formel Y-M umsetzt, in welcher

Y die in Anspruch 1 genannten Bedeutungen hat und

M Wasserstoff oder ein Metalläquivalent ist,

wobei eine Verbindung I mit Z = OH entsteht und, falls gewünscht, diese Verbindung I acyliert, alkyliert oder in eine Verbindung mit Z= F, Cl oder Br, überführt, und gegebenenfalls am Schwefel einer Thioethergruppe zum Sulfoxid oder Sulfon oxidiert,

und gegebenenfalls in das Salz mit einer physiologisch verträglichen Säure überführt.

5. Arzneimittel enthaltend eine wirksame Menge einer Verbindung I nach Anspruch 1.

6. Verwendung einer Verbindung I nach Anspruch 1 als Arzneimittel.

7. Verwendung einer Verbindung I nach Anspruch 1 zur Herstellung eines Medikaments mit antimykotischer Wirkung.

8. Fungizid, enthaltend eine wirksame Menge einer Verbindung I nach Anspruch 1.

9. Verwendung einer Verbindung I nach Anspruch 1 als Fungizid.

Patentansprüche Griechenland, Spanien und Österreich:

1. Verfahren zur Herstellung von Verbindungen der Formel I

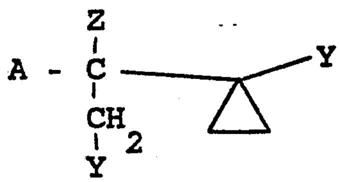

I,

worin bedeuten:

A t-Butyl, Phenyl, Biphenylyl, Phenoxyphenyl, Benzylphenyl, Benzyloxyphenyl, Phenylthiophenyl, Phenylsulfinylphenyl, Phenylsulfonylphenyl, Naphthyl, 1,2,3,4-Tetrahydronaphthyl, Indanyl, Fluorenyl, Thienyl, Furyl, Pyridyl, Isoxazolyl, Pyrazolyl, Benzofuryl, Benzothienyl,

wobei die genannten Ringsysteme unsubstituiert oder mit 1-3 Substituenten, die gleich oder verschieden sind, substituiert sind, welche F, Cl, Br, J, $(C_1-C_8)$-Alkyl, geradkettig oder verzweigt und unsubstituiert oder mit 1-9 F- oder Cl-Atomen substituiert, $(C_3-C_8)$-Cycloalkyl, $(C_1-C_8)$-Alkoxy, geradkettig oder verzweigt, und unsubstituiert oder mit 1-9 F- oder Cl-Atomen substituiert, $(C_3-C_8)$Cycloalkyl-, $(C_1-C_4)$-Alkyl, $(C_1-C_8)$-Alkylthio, $(C_1-C_8)$-Alkylsulfinyl- und -sulfonyl, $NO_2$, CN sind;

Y

1) 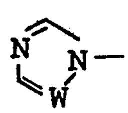

mit W= CH oder N

2) $- X - R^1$

mit X = -O-, -S-, >S=O, $>SO_2$ und

$R^1$ = $(C_1-C_{12})$-Alkyl (geradkettig oder verzweigt, unsubstituiert oder durch 1 bis 3 F-, Cl- oder Br-Atome oder $CH_3$-Gruppen substituiert), $(C_2-C_{20})$-Alkenyl (geradkettig oder verzweigt; ein- oder mehrfach ungesättigt, in Form der reinen e- oder z-Isomeren oder der e/z-Diastereomerengemische, unsubstituiert oder durch 1 bis 3 F-, Cl- oder Br-Atome oder $CH_3O$-Gruppen substituiert), $(C_2-C_{20})$-Alkinyl (geradkettig oder verzweigt; unsubstituiert oder durch 1 bis 3 F-, Cl- oder Br-Atome oder $CH_3O$-Gruppen substituiert), $(C_2-C_{20})$Alkeninyl (geradkettig oder verzweigt; ein- oder mehrfach ungesättigt, in Form der reinen e- oder z-Isomeren oder der e/z-Diastereomerengemische, unsubstituiert oder durch 1 bis 3 F-, Cl- oder Br-Atome oder $CH_3O$-Gruppen substituiert), $(C_3-C_8)$-Cycloalkyl, $(C_3-C_8)$Cycloalkyl-$(C_1-C_4)$-alkyl, Phenyl, Biphenylyl, Phenoxyphenyl, Phenylthiophenyl, Phenyl$(C_1-C_4)$-alkyl, Naphthyl, Biphenylyl$(C_1-C_4)$-alkyl, Phenylthiophenyl$(C_1-C_4)$-alkyl, Phenoxyphenyl-$(C_1-C_4)$alkyl, Naphthyl$(C_1-C_4)$-alkyl, Benzothiazol-2-yl, Benzimidazol-2-yl, N-$(C_1-C_4)$-Alkylbenzimidazol-2-yl, Benzyl, Pyridyl, Pyrid-2-yl-methyl, Pyrid-3-yl-methyl, Pyrid-4-yl-methyl, Pyrimidin-2-yl, Pyrimidin-2-yl-methyl, Pyrimidin-4-yl-methyl, Pyrimidin-5-yl-methyl, Furfuryl, Thien-2-yl, Thien-3-yl, Thien-2-yl-methyl, Thien-3-yl-methyl, Isoxazol-4-yl-methyl, Thiazol-2-yl-methyl, Thiazol-5-yl-methyl, Thiazol-5-yl-eth-2-yl, $(C_2-C_3)$-Alkyl-X-aryl (mit X wie vorstehend definiert und Aryl

gleich Phenyl, Benzyl, Thien-2-yl-methyl, Thien-3-yl-methyl), Benzothiazol-2-yl-methyl, Chinolin-yl-methyl, Pyridyl-phenyl-methyl, wobei die genannten Ringsysteme unsubstituiert oder durch 1, 2 oder 3 Substituenten, die gleich oder verschieden sind und jeweils F, Cl, Br, J, $CF_3$, $(C_1-C_4)$-Alkyl, $(C_1-C_4)$-Alkoxy bedeuten, substituiert sind; die Ringsysteme Pyridiminyl und Thienyl jedoch auch durch einen Phenylrest, der unsubstituiert oder seinerseits wie vorstehend angegeben substituiert ist,

3) 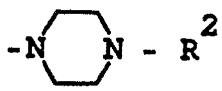

mit $R^2$= $(C_1-C_8)$-Alkyl, $(C_5-C_8)$-Cycloalkyl, Acetyl, Phenyl, Benzyl; wobei der Phenylkern jeweils unsubstituiert oder mit 1-3 F, Cl, Br, J, $CF_3$, $(C_1-C_4)$-Alkyl- oder $(C_1-C_4)$-Alkoxygruppen substituiert ist,

4) 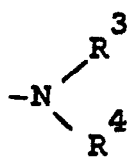 mit $R^3$ und $R^4$ gleich oder verschieden $(C_1-C_4)$Alkyl,

5) 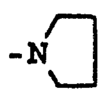 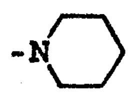 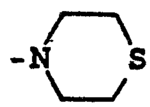 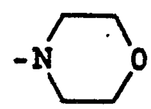

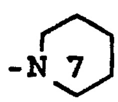 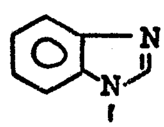 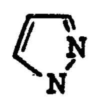

wobei mindestens eine der beiden Gruppen Y die Bedeutung von Y 1) hat;

Z = OH, $(C_1-C_4)$-Alkylcarbonyloxy, F, Cl, Br, $(C_1-C_4)$-Alkoxy, Benzyloxy, unsubstituiert oder 1-2fach substituiert mit F, Cl, Br, $CF_3$;

und ihrer Salze mit physiologisch verträglichen Säuren

mit Ausnahme derjenigen Verbindungen, in denen gleichzeitig

a) beide Y die Bedeutung von Y 1) mit W=N und

b) A die Bedeutung Phenyl, gegebenenfalls substituiert mit 1-3 Substituenten, die unabhängig voneinander ausgewählt sind aus F, Cl, Br, J, $CF_3$, $(C_1-C_4)$-Alkyl und $(C_1-C_4)$-Alkoxy, oder 5-Chlorpyrid-2-yl und

c) Z die Bedeutung wie oben angegeben

haben,

dadurch gekennzeichnet, daß man

a) eine Verbindung der Formel II

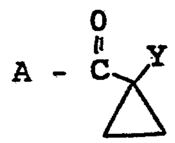

II,

in welcher A und Y die obengenannten Bedeutungen haben,

mit einem Schwefelylid der Formel III

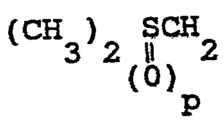

III,

worin p Null oder 1 bedeutet,

zu einer Verbindung der Formel IV

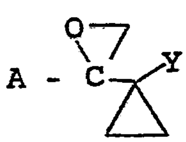

IV

umsetzt,

und anschließend die Verbindung IV mit einem Nukleophil der Formel Y-M umsetzt, in welcher

Y die obengenannte Bedeutung hat und

M Wasserstoff oder ein Metalläquivalent ist,

wobei eine Verbindung I mit Z= OH entsteht, und, falls gewünscht, diese Verbindung I acyliert, alkyliert oder in eine Verbindung mit Z= F, Cl oder Br überführt, und gegebenenfalls am Schwefel einer Thioethergruppe zum Sulfoxid oder Sulfon oxidiert;

b) eine Verbindung der Formel II

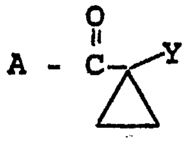 II,

in welcher A und Y die obengenannten Bedeutungen haben, mit einem Phosphorylid zu einer Verbindung der Formel V

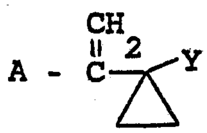 V

umsetzt,
und anschließend die Verbindung V mit einem organischen Peroxid zu einer Verbindung der Formel IV

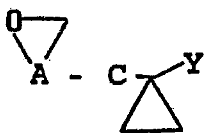 IV

umsetzt,
und anschließend die Verbindung IV mit einem Nucleophil der Formel Y-M umsetzt, in welcher
Y die obengenannten Bedeutungen hat und
M Wasserstoff oder ein Metalläquivalent ist,
wobei eine Verbindung I mit Z = OH entsteht und, falls gewünscht, diese Verbindung I acyliert, alkyliert oder in eine Verbindung mit Z= F, Cl oder Br, überführt, und gegebenenfalls am Schwefel einer Thioethergruppe zum Sulfoxid oder Sulfon oxidiert,
und gegebenenfalls in das Salz mit einer physiologisch verträglichen Säure überführt.

2. Verfahren nach Anspruch 1, dadurch gekennzeichnet, daß mindestens einer der Substituenten folgende Bedeutung hat:

A: Phenyl, Biphenylyl, 1,2,3,4-Tetrahydronaphthyl, Thienyl, Indanyl, jeweils unsubstituiert oder im Ring mit ein oder zwei Substituenten, die gleich oder verschieden sind und jeweils F, Cl, Br, $CF_3$, $(C_1-C_4)$-Alkyl oder $(C_1-C_4)$-Alkoxy bedeuten, substituiert sind,

Y: 1) 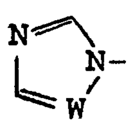 mit W= CH oder N

2) $-X-R^1$ mit

$X = O, S$

$R^1 = (C_1-C_{15})$-Alkyl, geradkettig oder verzweigt, $(C_2-C_{15})$-Alkenyl, geradkettig oder verzweigt, ein- oder mehrfach ungesättigt Phenyl, Biphenylyl, Phenoxyphenyl, Phenylthiophenyl, Phenyl-$(C_1-C_2)$alkyl, Naphthyl, Biphenylyl$(C_1-C_2)$alkyl, Naphthyl$(C_1-C_2)$alkyl, Benzothiazol-2-yl, Benzimidazol-2-yl, Furfuryl, Thien-2-yl-methyl, Thieny-3-yl-methyl, Isoxazol-4-yl-methyl, Pyrid-3-yl-methyl, Pyrid-4-yl-methyl, Ethyl-thio-aryl mit Aryl gleich Phenyl, Benzyl, Thien-2-ylmethyl, Thien-3-yl-methyl, wobei die genannten Ringsysteme unsubstituiert oder durch 1, 2 oder 3 Substituenten, die gleich oder verschieden sind und jeweils F, Cl, Br, $CF_3$, Methyl, Methoxy-Gruppen bedeuten, substituiert sind,

3.) 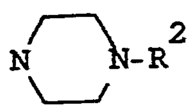 mit

$R^2$ = Phenyl, Benzyl, jeweils unsubstituiert oder mit 1 oder 2 F, Cl, Br, $CF_3$, Methyl oder Methoxy-Gruppen substituiert,

Z = OH, $OCH_3$, F, Cl
wobei mindestens eine der beiden Gruppen Y die Bedeutung von Y 1) hat.

3. Verfahren nach Anspruch 1, dadurch gekennzeichnet, daß mindestens einer der Substituenten folgende Bedeutung hat:

A Phenyl, Thienyl, jeweils unsubstituiert oder substituiert durch 1 oder 2 F- oder Cl-Atome, Methyl, Methoxy

Y 1) 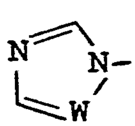 mit W = CH oder N

2) $-X-R^1$ mit $X = O, S$
$R^1 =$ $(C_1-C_{12})$-Alkyl (geradkettig oder verzweigt), Geranyl, Neryl, Phenyl, Benzyl, Naphthyl, Thien-2-yl-methyl Thien-3-yl-methyl, Isoxazol-4-yl-methyl, Pyrid-3-yl-methyl, Pyrid-4-yl-methyl, jeweils unsubstituiert oder 1- oder 2-fach substituiert durch F, Cl, Methyl, Methoxy

Z = OH
wobei mindestens eine der beiden Gruppen Y die Bedeutung von Y 1) hat.

4. Verfahren zum Herstellen eines Arzneimittels, dadurch gekennzeichnet, daß man eine wirksame Menge einer nach Anspruch 1 erhaltenen Verbindung mit pharmazeutisch üblichen Trägerstoffen mischt.

5. Verwendung einer nach Anspruch 1 erhaltenen Verbindung I als Arzneimittel.

6. Verwendung einer nach Anspruch 1 erhaltenen Verbindung I zur Herstellung eines Medikaments mit antimykotischer Wirkung.

7. Verwendung einer nach Anspruch 1 erhaltenen Verbindung I zur Herstellung eines Mittels mit fungizider Wirkung.